(19) Europäisches Patentamt
European Patent Office
Office européen des brevets

(11) EP 2 691 376 B1

(12) **EUROPÄISCHE PATENTSCHRIFT**

(45) Veröffentlichungstag und Bekanntmachung des Hinweises auf die Patenterteilung:
**09.12.2015 Patentblatt 2015/50**

(21) Anmeldenummer: **12711629.1**

(22) Anmeldetag: **28.03.2012**

(51) Int Cl.:
***C07D 239/34*** (2006.01) ***C07D 239/80*** (2006.01)
***A61P 7/00*** (2006.01) ***A61K 31/26*** (2006.01)
***A61K 33/26*** (2006.01) ***A61K 31/513*** (2006.01)

(86) Internationale Anmeldenummer:
**PCT/EP2012/055512**

(87) Internationale Veröffentlichungsnummer:
**WO 2012/130882 (04.10.2012 Gazette 2012/40)**

(54) **FE(III)-KOMPLEXVERBINDUNGEN ZUR BEHANDLUNG UND PROPHYLAXE VON EISENMANGELERSCHEINUNGEN UND EISENMANGELANÄMIEN**

FE(III) COMPLEXES FOR THE TREATMENT AND PROPHYLAXIS OF IRON DEFICIENCY SYMPTOMS AND IRON DEFICIENCY ANAEMIAS

COMPOSÉS À BASE DE COMPLEXES DE FE(III) POUR LE TRAITEMENT ET LA PRÉVENTION DE CARENCES EN FER ET D'ANÉMIES DUES À UNE CARENCE EN FER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.03.2011 EP 11160151**

(43) Veröffentlichungstag der Anmeldung:
**05.02.2014 Patentblatt 2014/06**

(73) Patentinhaber: **Vifor (International) AG**
**9001 St. Gallen (CH)**

(72) Erfinder:
- **BARK, Thomas**
  **8050 Zürich (CH)**
- **BUHR, Wilm**
  **78465 Konstanz (DE)**
- **BURCKHARDT, Susanna**
  **8049 Zürich (CH)**
- **BURGERT, Michael**
  **88045 Friedrichshafen (DE)**
- **CANCLINI, Camillo**
  **9000 St. Gallen (CH)**
- **DÜRRENBERGER, Franz**
  **4143 Dornach (CH)**
- **FUNK, Felix**
  **8404 Winterthur (CH)**
- **GEISSER, Peter Otto**
  **9014 St. Gallen (CH)**
- **KALOGERAKIS, Aris**
  **8400 Winterthur (CH)**
- **MAYER, Simona**
  **9055 Bühler (CH)**
- **PHILIPP, Erik**
  **9320 Arbon (CH)**
- **REIM, Stefan**
  **8404 Winterthur (CH)**
- **SIEBER, Diana**
  **9030 Abtwil (CH)**
- **SCHMITT, Jörg**
  **9425 Thal (CH)**
- **SCHWARZ, Katrin**
  **9000 St. Gallen (CH)**

(74) Vertreter: **Gille Hrabal**
**Brucknerstrasse 20**
**40593 Düsseldorf (DE)**

(56) Entgegenhaltungen:
**EP-A2- 0 138 420**

- **J OHKANDA ET AL: "N-Hydroxyamide-Containing Heterocycles. Part 2. Synthesis and Iron(III) Complex-Forming Tendency of 1-Hydroxy-2(1H)-pyrimidinone and -pyrazinone", BULLETIN OF THE CHEMICAL SOCIETY OF JAPAN, Bd. 66, 1. Januar 1993 (1993-01-01), Seiten 841-847, XP055000884,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 691 376 B1

Printed by Jouve, 75001 PARIS (FR)

## Beschreibung

### Einleitung:

Die Erfindung betrifft Eisen(III)-pyrimidin-2-ol-1-oxid-Komplexverbindungen und deren sie umfassende pharmazeutische Zusammensetzungen zur Verwendung als Arzneimittel, insbesondere zur Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.

Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.

Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.

Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117,2004,285-297.)

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoesestimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurückgeführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik sind Eisenkomplex-Verbindungen für die Behandlung von Eisenmangelzuständen bekannt.

Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).

Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009,22,701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

In der Patent-Literatur sind auch Hydroxypyron- und Hydroxypyridon-Fe-Komplexverbindungen beschrieben (EP159194, EP138420, EP107458). Analog hierzu sind auch die entsprechenden 5-Ringsysteme, die Hydroxyfuranon-Fe-Komplexverbindungen beschrieben (WO2006037449). Insbesondere die Hydroxypyridon-Fe-Komplexverbindungen weisen jedoch eine vergleichsweise geringe Wasserlöslichkeit auf, weshalb sie insbesondere für die orale Verabreichung weniger geeignet sind. Des weiteren weisen die Hydroxypyridon-Fe-Komplexverbindungen eine vergleichsweise geringe Eisenutilisation auf.

Ausserdem sind in der Literatur Eisen-Cyclopentadienyl-Komplexverbindungen beschrieben (GB842637).

Im weiteren sind in der Literatur auch 1-Hydroxy-4,6-dimethyl-2(1 *H)*-pyrimidinone als Fe(III)-Liganden beschrieben worden (Bull. Chem. Soc. Jpn., 66, 841-841(1993)), und als mögliche Struktur eines entsprechenden Eisen(III)-Komplexes wird folgende Struktur angegeben:

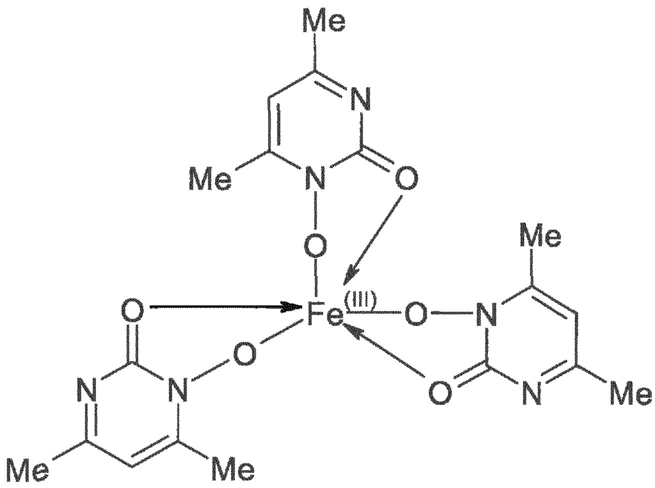

(s. auch "Reviews On Heteroatom Chemistry", Vol. 18, 1998, Seite 87 bis 118 derselben Autoren). Eine Charakterisierung dieses Komplexes erfolgte allerdings nur in Lösung. Eine feste Form dieses Komplexes wird nicht offenbart. Auch werden die Eisen-Komplexverbindungen nicht als Arzneimittel, insbesondere für die Behandlung von Eisenmangelzuständen vorgeschlagen oder verwendet. Dieselben Autoren schlagen lediglich die Verwendung sechszähniger 1-Hydroxy-1H-pyrimidin-2-on-Verbindungen als Eisenmaskierungsmittel zur Behandlung von Eisenüberladungszuständen, wie Thalassämie, vor (J. Org. Chem. 1997, 62, 3618-3624). Dabei könnte durch die Verabreichung der Hydroxy-pyrimidinon-Verbindungen dem Körper zur Behandlung der Thalassämie Eisen entzogen werden - also nicht Eisen zugeführt werden - wie bei der erfindungsgemäß durchgeführten Behandlung der Eisenmangelanämie durch Verabreichung von Eisen-Komplexverbindungen.

J. Am. Chem. Soc. 1985, 107, 6540-6546 beschreibt vierzähnige 1-Hydroxy-1H-pyridin-2-on-Verbindungen als Liganden und eine zweikernige Eisenkomplexverbindung damit. Die Möglichkeit den Liganden zur Eisenmaskierung zu verwenden wird ebenfalls erwähnt. Ähnlich offenbart Inorganica Chimica Acta, 135 (1987) 145-150 die Verwendung von 1-Hydroxy-1H-pyridin-2-onen als Mittel zur Eisenmaskierung.

Ein anderer wichtiger Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)lonen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und wird in der Literatur weitgehend als Ursache für die hohe Unverträglichkeit angesehen und als Oxidativer Stress bezeichnet.

Eisen(III)-1-Hydroxy-1H-pyrimidin-2-on- bzw. Pyrimidin-2-ol 1-oxid-Komplexverbindungen sind im Stand der Technik somit bisher weder als Arzneimittel noch insbesondere zur Verwendung in der Behandlung und/oder zur Prophylaxe von Eisenmangelerscheinungen und Eisenmangelanämien beschrieben worden.

**Aufgabenstellung:**

Die Aufgabe der vorliegenden Erfindung bestand darin, neue therapeutisch wirksame Verbindungen zu entwickeln, die für eine effektive Therapie zur bevorzugt oralen Behandlung von Eisenmangelerscheinungen und Eisenmangelanämien eingesetzt werden können. Dabei sollten diese Eisenkomplexe gleichzeitig deutlich weniger Nebenwirkungen aufzeigen, als die klassisch verwendeten Fe(II)-Salze. Ausserdem sollten diese Eisenkomplexe im Unterschied zu den bekannten polymeren Eisenkomplexverbindungen möglichst eine definierte Struktur (Stöchiometrie) aufweisen und durch einfache Synthesewege darstellbar sein. Des weiteren sollten die Verbindungen bei oraler Verabreichung zu einer hohen Utilisationsrate des Eisens führen, was durch eine gute Wasserlöslichkeit unterstützt wird. Schließlich sollten die EisenkomplexVerbindungen über eine möglichst geringe Toxizität verfügen und somit in möglichst hohen Dosierungen verabreichbar sein. Dieses Ziel wurde durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Die neuen Verbindungen sollten aufgrund ihrer

Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue Fe(III)-Komplexverbindungen mit nicht zu grossem Molekulargewicht, mittlerer Lipophilie und optimaler Komplexstabilität.

**Beschreibung der Erfindung:**

Die Erfinder fanden überraschend, dass Fe(III)-Komplexverbindungen mit Pyrimidin-2-ol 1-oxid-Liganden sich für die oben beschriebenen Anforderungen besonders eignen. Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik bekannten Fe Salzen auf. Die Komplexverbindungen zeigen in unterschiedlichen pH-Wert-Bereichen gute Stabilitäten und vergleichsweise gute Löslichkeiten. Des Weiteren verfügen die Eisenkomplex-Verbindungen über eine sehr geringe Toxizität und können somit in hohen Dosierungen ohne Nebenwirkungen verabreicht werden. Die Komplexverbindungen lassen sich schließlich gut herstellen und eignen sich optimal zur Formulierung von Arzneimitteln, insbesondere zur oralen Verabreichung.

Gegenstand der Erfindung sind somit Eisen(III)-pyrimidin-2-ol 1-oxid-Komplexverbindungen oder deren pharmazeutisch verträgliche Salze zur Verwendung als Arzneimittel bzw. gleichbedeutend zur Anwendung in einem Verfahren zur therapeutischen Behandlung des menschlichen Körpers.

Die erfindungsgemäß verwendeten Eisen(III)-pyrimidin-2-ol 1-oxid-Komplexverbindungen schließen insbesondere solche Verbindungen ein, die folgendes Strukturelement aufweisen:

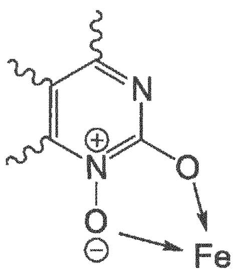

worin

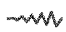

jeweils ein die freie Valenz absättigender Substituent des Liganden ist und die Pfeile jeweils koordinative Bindungen zum Eisenatom darstellen. Die Begriffe

- "Pyrimidin-2-ol 1-oxid",
- "Pyrimidin-2-ol 1-oxid-Verbindungen" oder
- "Pyrimidin-2-ol 1-oxid-"-Liganden

schließen somit erfindungsgemäß sowohl die entsprechenden Hydroxy-Ausgangverbindungen

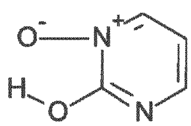

als auch die entsprechenden deprotonierten Liganden

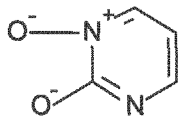 bzw. 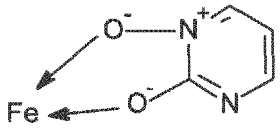 ,

ein, welche in den entsprechenden Eisen(III)-Komplexverbindungen vorliegen.

Desweiteren schließen die genannten Begriffe erfindungsgemäß nicht nur den entsprechenden Grundkörper:

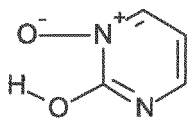

bzw. die durch Deprotonierung aus der zugrundeliegenden Hydroxyverbindung

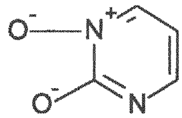

hervorgegangene Ligand-Verbindung ein, sondern auch deren am Pyrimidinring substituierte Vertreter, welche aus dem Austausch eines oder mehrerer Wasserstoffatome am Pyrimidinring gegen andere Substituenten resultieren. Die genannten Begriffe bezeichnen somit erfindungsgemäß die Gesamtheit der Klasse der "Pyrimidin-2-ol 1-oxid"-Verbindungen bzw. der deprotonierten Liganden, einschließlich der am Pyrimidinring substituierten Vertreter.

Ein (deprotonierter) Pyrimidin-2-ol 1-oxid-Ligand wie er erfindungsgemäß verwendet wird, trägt somit formal eine negative Ladung. Das bedeutet, daß bei drei Liganden je Eisenatom das Eisenatom formal die Oxidationsstufe +3 besitzt. Es ist für den Fachmann klar, dass die gezeigten Formeln nur eine mögliche mesomere Grenzformel darstellen, und dass mehrere mesomere Grenzformeln existieren bzw. eine Delokalisierung der Elektronen im Liganden bzw. der Eisen-Komplexverbindung vorliegt, wie nachfolgend noch schematisch gezeigt wird.

In den erfindungsgemäßen Eisen(III)-pyrimidin-2-ol 1-oxid-Komplexverbindungen beträgt die Koordinationszahl der Eisenatome im Allgemeinen sechs (6), wobei im Allgemeinen eine oktaedrische Anordnung der koordinierenden Atome vorliegt.

Erfindungsgemäß sind weiterhin ein- oder mehrkernige Eisen(III)-pyrimidin-2-ol 1-oxid-Komplexverbindungen umfasst, in denen ein oder mehrere (wie 2, 3 oder 4) Eisenatome vorhanden sind.

Im allgemeinen können 1-4 Eisenatome und 2-10 Liganden in den Eisen(III)-pyrimidin-2-ol 1-oxid-Komplexverbindungen vorliegen. Bevorzugt sind jedoch einkernige Eisen(III)-pyrimidin-2-ol 1-oxid-Komplexverbindungen mit mindestens einem bevorzugt 3, bevorzugt zweizähnigen Pyrimidin-2-ol 1-oxid-Liganden. Bevorzugt sind jedoch einkernige Eisen(III)-pyrimidin-2-ol 1-oxid-Komplexverbindungen, in denen ein (1) zentrales Eisenatom und drei (3) Pyrimidin-2-ol 1-oxid-Liganden vorliegen.

Die erfindungsgemäßen Eisen(III)-pyrimidin-2-ol 1-oxid-Komplexverbindungen liegen im Allgemeinen in neutraler Form vor. Es sind jedoch auch salzartige Eisen(III)-pyrimidin-2-ol 1-oxid-Komplexverbindungen eingeschlossen, in denen der Komplex eine positive oder negative Ladung aufweist, die insbesondere durch pharmakologisch verträgliche, im wesentlichen nicht-koordinierende Anionen (wie insbesondere Halogenide, wie Chlorid) oder Kationen (wie insbesondere Alkali- oder Erdalkalimetallionen) ausgeglichen wird.

Die erfindungsgemäßen Eisen(III)-pyrimidin-2-ol 1-oxid-Komplexverbindungen schließen insbesondere solche Komplexverbindungen ein, die mindestens einen, bevorzugt zweizähnigen Pyrimidin-2-ol 1-oxid-Liganden der Formel

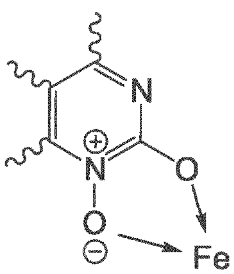

aufweisen, worin

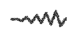

jeweils ein die freie Valenz absättigender Substituent des Liganden ist, der, wie vorstehend gezeigt, an ein oder auch an zwei verschiedene Eisenatome im Sinne einer Verbrückung gebunden sein kann.

Bevorzugt sind Eisen(III)-pyrimidin-2-ol 1-oxid-Komplexverbindungen, die ausschließlich, bevorzugt zweizähnige Pyrimidin-2-ol 1-oxid-Liganden aufweisen, welche gleich oder verschieden sein können. Besonders bevorzugt sind weiterhin Eisen(III)-pyrimidin-2-ol 1-oxid-Komplexverbindungen, die ausschließlich die gleichen Pyrimidin-2-ol 1-oxid-Liganden aufweisen, und ganz besonders bevorzugt sind Tris(pyrimidin-2-ol 1-oxid)-eisen(III)-Verbindungen.

Bevorzugt liegt das Molekulargewicht der erfindungsgemäßen Eisen(III)-pyrimidin-2-ol 1-oxid-Komplexverbindungen bei weniger als 1000 g/mol, noch bevorzugter bei weniger als 850 g/mol, noch bevorzugter bei weniger als 700 g/mol (jeweils bestimmt aus der Strukturformel).

Die erfindungsgemäßen Eisen(III)-Komplexverbindungen enthalten mindestens einen, bevorzugt drei gleiche oder verschiedene, bevorzugt gleiche Liganden der Formel (I):

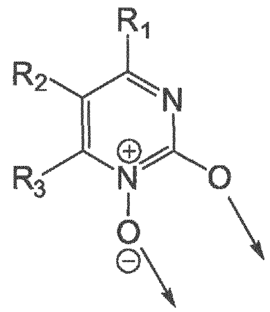

(I)

worin

die Pfeile jeweils eine koordinative Bindung zu einem oder verschiedenen Eisenatomen darstellen, und

$R_1$, $R_2$, $R_3$ gleich oder verschieden sein können und ausgewählt werden aus der Gruppe, die besteht aus:

- Wasserstoff,
- gegebenenfalls substituiertem Alkyl,
- Halogen,
- gegebenenfalls substituiertem Alkoxy,
- gegebenenfalls substituiertem Aryl,
- gegebenenfalls substituiertem Alkoxycarbonyl,
- gegebenenfalls substituiertem Amino, und
- gegebenenfalls substituiertem Aminocarbonyl ist, oder

$R_1$ und $R_2$ oder $R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann, oder pharmazeutisch verträgliche Salze davon.

Die vorstehend erwähnte Ringbildung der Substituenten $R_1$ und $R_2$ oder $R_2$ und $R_3$ ist schematisch in den folgenden Formeln gezeigt:

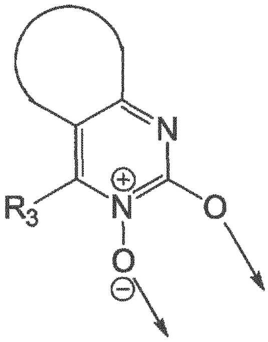 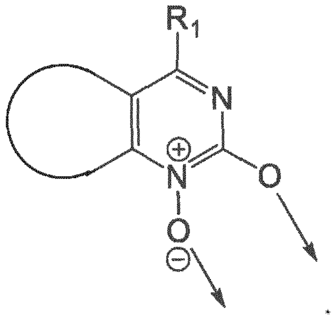

In einer bevorzugten Ausführungsform der Erfindung betrifft diese Eisen(III)-Komplexverbindungen enthaltend mindestens einen Liganden der Formel (I)

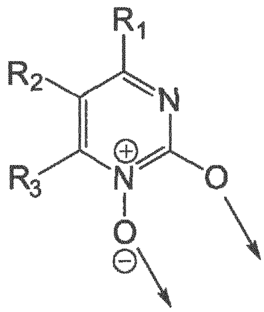

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl,
- Halogen,
- gegebenenfalls substituiertem Alkoxy,
- gegebenenfalls substituiertem Aryl,
- gegebenenfalls substituiertem Alkoxycarbonyl, und
- gegebenenfalls substituiertem Aminocarbonyl ist, oder

$R_1$ und $R_2$ oder $R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann,
oder pharmazeutisch verträgliche Salze davon.

In einer bevorzugten Ausführungsform der Erfindung betrifft diese Eisen(III)-Komplexverbindungen enthaltend mindestens einen Liganden der Formel (I):

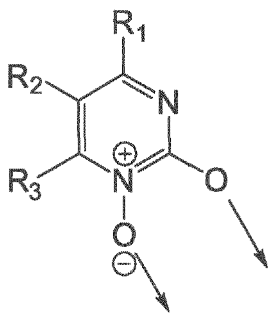

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl, und
- Halogen.

Gegebenenfalls substituiertes Alkyl schließt im Rahmen der gesamten Erfindung insbesondere für die Substituenten $R_1$ bis $R_3$ bevorzugt ein:

Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, wobei diese Alkylgruppen gegebenenfalls substituiert sein können.

Die genannten Alkylgruppen können unsubstituiert oder substituiert, bevorzugt mit 1 bis 3 Substituenten, sein. Diese Substituenten an den Alkylgruppen werden bevorzugt aus der Gruppe ausgewählt, die besteht aus: Hydroxy, gegebenenfalls substituiertem Aryl, insbesondere wie nachstehend definiert, gegebenenfalls substituiertem Heteroaryl, insbesondere wie nachstehend definiert, gegebenenfalls substituiertem Alkoxy insbesondere wie nachstehend definiert, gegebenenfalls substituiertem Alkoxycarbonyl, insbesondere wie nachstehend definiert, gegebenenfalls substituiertem Acyl, insbesondere wie nachstehend definiert, Halogen, insbesondere wie nachstehend definiert, gegebenenfalls substituierten Amino, insbesondere wie nachstehend definiert, gegebenenfalls substituierten Aminocarbonyl, insbesondere wie nachstehend definiert, und Cyano.

Halogen schließt hier und im Rahmen der vorliegende Erfindung Fluor, Chlor, Brom und lod, bevorzugt Fluor oder Chlor, ein.

In den vorstehend definierten Alkylgruppen können des weiteren gegebenenfalls ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere, bevorzugt 1 bis 3, noch bevorzugter eine (1) Methylengruppe ($-CH_2-$) in den Alkylresten durch -NH-, $-NR_4-$, -O- oder -S- ersetzt sein können, worin $R_4$ gegebenenfalls substituiertes Alkyl, wie vorstehend definiert, bevorzugt gegebenenfalls mit 1 bis 3 Substituenten, wie Fluor, Chlor, Hydroxy oder Alkoxy, substituiertes C1-C6 Alkyl, wie Methyl oder Ethyl ist.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1-Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen. Am meisten bevorzugt sind Methyl, Ethyl, n-Propyl, Isopropyl, sek.-Butyl und n-Butyl.

Beispiele von Alkylgruppen, die durch Austausch mit einer oder mehreren heteroanalogen Gruppe, wie -O-, -S-, -NH- oder $-N(R_4)-$ hervorgehen, sind bevorzugt solche, in denen eine oder mehrere Methylengruppen ($-CH_2-$) durch -O- unter Bildung einer Ethergruppe ersetzt sind, wie Methoxymethyl, Ethoxymethyl, 2-Methoxyethyl usw. Daher schließt die Definition von Alkyl auch beispielsweise Alkoxyalkylgruppen, wie nachstehend definiert, ein, welche durch Austausch einer Methylengruppe durch -O- aus den genannten Alkylgruppen hervorgehen. Sind erfindungsgemäß darüber hinaus Alkoxygruppen als Substituenten von Alkyl zugelassen, können auch auf diese Weise mehrere Ethergruppen gebildet werden (wie zum Beispiel eine $-CH_2-O-CH_2-OCH_3$-Gruppe). Erfindungsgemäß sind somit auch Polyethergruppen von der Definition von Alkyl umfasst.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw. ein. Bevorzugt sind eine Cyclopropylgruppe, eine Cyclobutylgruppe, eine Cyclopentylgruppe und eine Cyclohexylgruppe.

Die Cycloalkylreste können gegebenenfalls substituiert sein, bevorzugt mit 1 bis 2 Substituenten, wie Hydroxy oder C1-C6-Alkoxycarbonyl.

Die Definition der gegebenenfalls substituierten Alkylgruppen schließt auch Alkylgruppen ein, welche durch vorstehend definierte Cycloalkylgruppen substituiert sind, wie beispielsweise Cyclopropylmethyl, Cyclobutylmethyl, Cyclopentylmethyl oder Cyclohexylmethyl.

Heterocyclische Alkylreste sind erfindungsgemäß insbesondere solche, die durch Austausch von Methylen durch Heteroanaloge Gruppen aus Cycloalkyl gebildet werden, und schließen beispielsweise gesättigte 5- oder 6-gliedrige heterocyclische Reste ein, welche über ein Kohlenstoffatom oder ein Stickstoffatom angebunden sein können, und welche bevorzugt 1 bis 3, bevorzugt 2 Heteroatome wie insbesondere O, N aufweisen können, wie Tetrahydrofuryl, Azetidin-1-yl, substituiertes Azetidinyl, wie 3-Hydroxyazetidin-1-yl, Pyrrolidinyl, wie Pyrrolidin-1-yl, substituiertes Pyrrolidinyl, wie 3-Hydroxypyrrolidin-1-yl, 2-Hydroxypyrrolidin-1-yl, 2-Methoxycarbonylpyrrolidin-1-yl, 2-Ethoxycarbonylpyrrolidin-1-yl, 2-Methoxypyrrolidin-1-yl, 2-Ethoxypyrrolidin-1-yl, 3-Methoxycarbonylpyrrolidin-1-yl, 3-Ethoxycarbonylpyrrolidin-1-yl, 3-Methoxypyrrolidin-1-yl, 3-Ethoxypyrrolidin-1-yl, Piperidinyl, wie Piperidin-1-yl, Piperidin-4-yl, substituiertes Piperidinyl, wie 4-Methyl-1-piperidyl, 4-Hydroxy-1-piperidyl, 4-Methoxy-1-piperidyl, 4-Ethoxy-1-piperidyl, 4-Methoxycarbonyl-1-piperidyl, 4-Ethoxycarbonyl-1-piperidyl, 4-Carboxy-1-piperidyl, 4-Acetyl-1-piperidyl, 4-Formyl-1-piperidyl, 1-Methyl-4-piperidyl, 4-Hydroxy-2,2,6,6-tetramethyl-1-piperidyl, 4-(Dimethylamino)-1-piperidyl, 4-(Diethylamino)-1-piperidyl, 4-Amino-1-piperidyl, 2-(Hydroxymethyl)-1-piperidyl, 3-(Hydroxymethyl)-1-piperidyl, 4-(Hydroxymethyl)-1-piperidyl, 2-Hydroxy-1-piperidyl, 3-Hydroxy-1-piperidyl, 4-Hydroxy-1-piperidyl, Morpholin-4-yl, substituiertes Morpholinyl, wie 2,6-Dimethylmorpholin-4-yl, Piperazinyl, wie Piperazin-1-yl, substituiertes Piperazinyl, wie 4-Methylpiperazin-1-yl, 4-Ethylpiperazin-1-yl, 4-Ethoxycarbonylpiperazin-1-yl, 4-Methoxycarbonylpiperazin-1-yl, oder Tetrahydropyranyl, wie Tetrahydropyran-4-yl, und welche gegebenenfalls mit aromatischen Ringen kondensiert sein können, und welche gegebenenfalls substituiert sein können, wie mit 1 bis 2 Substituenten, wie Hydroxy, Halogen, C1-C6-Alkyl etc. Die Definition der gegebenenfalls substituierten Alkylgruppen schließt somit auch Alkylgruppen ein, welche durch vorstehend definierte heterocyclische Gruppen substituiert sind, wie beispielsweise 3-(1-Piperidyl)propyl, 3-Pyrrolidin-1-ylpropyl, 3-Morpholinopropyl, 2-Morpholinoethyl, 2-Tetrahydropyran-4-ylethyl, 3-Tetrahydropyran-4-ylpropyl, 3-(Azetidin-1-yl)propyl etc..

Beispiele eines mit Halogen substituierten linearen oder verzweigten Alkylrestes mit 1 bis 8 bevorzugt 1 bis 6 Kohlenstoffatomen schließen insbesondere ein: eine Fluormethylgruppe, eine Difluormethylgruppe, eine Trifluormethylgruppe, eine Chlormethylgruppe, eine Dichlormethylgruppe, eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe, eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe, eine 1-Brompropylgruppe, eine 2-Fluorpropylgruppe, eine 2-Chlorpropylgruppe, eine 2-Brompropylgruppe, eine 3-Fluorpropylgruppe, eine 3-Chlorpropylgruppe, eine 3-Brompropylgruppe, eine 1,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe, eine 2-Brompentylgruppe, eine 5-Fluorpentylgruppe, eine 5-Chlorpentylgruppe, eine 5-Brompentylgruppe, eine Perfluorpentylgruppe, eine 2-Fluorhexylgruppe, eine 2-Chlorhexylgruppe, eine 2-Bromhexylgruppe, eine 6-Fluorhexylgruppe, eine 6-Chlorhexylgruppe, eine 6-Bromhexylgruppe, eine Perfluorhexylgruppe, eine 2-Fluorheptylgruppe, eine 2-Chlorheptylgruppe, eine 2-Bromheptylgruppe, eine 7-Fluorheptylgruppe, eine 7-Chlorheptylgruppe, eine 7-Bromheptylgruppe, eine Perfluorheptylgruppe, usw.

Beispiele eines mit Hydroxy substituierten Alkylrestes schließen die oben genannten Alkylreste ein, die 1 bis 3 Hydroxylreste aufweisen, wie zum Beispiel Hydroxymethyl, 2-Hydroxyethyl, 3-Hydroxypropyl, 4-Hydroxybutyl, 5-Hydroxypentyl, 6-Hydroxyhexyl.

Gegebenenfalls substituiertes Aryl schließt erfindungsgemäß bevorzugt aromatische Kohlenwasserstoffreste mit 6 bis 14 Kohlenstoffatomen (ohne Heteroatome im aromatischen Ringsystem) ein, wie beispielweise: Phenyl, Naphthyl, Phenanthrenyl und Anthracenyl. Die genannten aromatischen Reste können unsubstituiert oder substituiert sein. Im Falle der Substitution weisen sie bevorzugt einen oder mehrere, bevorzugt einen (1) oder zwei (2) Substituenten auf, wie insbesondere Halogen, Hydroxy, Alkyl, Alkoxy, jeweils wie vorstehend oder nachstehend erläutert. Bevorzugter aromatischer Rest ist Phenyl. Bevorzugtes mit einer aromatischen Gruppe substituiertes Alkyl (Arylalkyl) ist Benzyl.

Gegebenenfalls substituiertes Aryl schließt erfindungsgemäß weiterhin gegebenenfalls substituiertes Heteroaryl also heteroaromatische Reste ein, wie beispielsweise: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. 5- oder 6-gliedrige aromatische Heterocyclen wie z.B. Pyridyl, Pyridyl-N-oxid, Pyrimidyl, Pyridazinyl, Furyl und Thienyl sind bevorzugt. Die genannten heteroaromatischen Reste können unsubstituiert oder substituiert sein. Im Falle der Substitution weisen sie bevorzugt einen oder mehrere, bevorzugt einen (1) oder (2) Substituenten auf, insbesondere Halogen, Hydroxy, Alkyl, Alkoxy, jeweils wie vorstehend

oder nachstehend erläutert. Bevorzugte Beispiele einer mit einer heteroaromatischen Gruppe substituiertes Alkyl (Hetarylalkyl) sind Methyl, Ethyl oder Propyl jeweils substituiert mit einer heteroaromatischen Gruppe, wie Thienylmethyl, Pyridylmethyl etc.

Gegebenenfalls substituiertes Alkoxy (RO-) leitet sich formal durch Hinzufügen eines Sauerstoffatom zu einem der vorstehend erwähnten gegebenenfalls substituierten Alkylreste ab, und schließt erfindungsgemäß beispielsweise lineare oder verzweigte Alkoxyreste bevorzugt mit bis zu 6 Kohlenstoffatomen ein, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw. ein. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, usw. Die Alkoxygruppen können gegebenenfalls substituiert sein, wie beispielsweise mit den für Alkyl genannten möglichen Substituenten.

Bevorzugtes Alkoxy ist Methoxy, Ethoxy, n-Propoxy, n-Butoxy etc..

Gegebenenfalls substituierte Alkoxycarbonyl (RO-CO-)-Gruppen leiten sich aus den vorstehend definierten Alkylgruppen formal durch Hinzufügen eines -O-C(O)-Restes ab unter Bildung eines gegebenenfalls substituierten Alkyloxycarbonyl-Restes ab. Insoweit kann auf die Definition der vorstehend beschriebenen Alkylgruppen verwiesen werden. Alternativ leiten sich gegebenenfalls substituierte Alkoxycarbonyl (RO-CO-)-Gruppen aus den vorstehend genannten Alkoxygruppen entsprechend formal durch Hinzufügen einer Carbonylgruppe ab. Bevorzugte Alkoxycarbonyl-Gruppen weisen bis zu 6 Kohlenstoffatome auf und schließen beispielsweise ein: Methoxycarbonyl, Ethoxycarbonyl, n-Propoxycarbonyl, n-Butoxycarbonyl, tert.-Butoxycarbonyl etc., welche sämtlich wie vorstehend definierte Alkylgruppen substituiert sein können.

Gegebenenfalls substituiertes Amino schließt erfindungsgemäß bevorzugt ein: Amino ($-NH_2$), gegebenenfalls substituiertes Mono- oder Dialkylamino (RHN-, $(R)_2N$-) wobei hinsichtlich der Definition von gegebenenfalls substituiertem Alkyl auf vorstehende Definition verwiesen werden kann. Ferner sind gegebenenfalls substituierte Mono- oder Diarylamino-Reste eingeschlossen oder gemischte gegebenenfalls substituierte Alkylarylamino-Reste, wobei hinsichtlich der Definition von gegebenenfalls substituiertem Alkyl oder Aryl auf vorstehende Definition verwiesen werden kann. Derartige Gruppen schließen beispielsweise Methylamino, Dimethylamino, Ethylamino, Hydroxyethylamino, wie 2-Hydroxyethylamino, Diethylamino, Phenylamino, Methylphenylamino etc. ein. Besonders bevorzugt ist Ethylamino. Gegebenenfalls substituiertes Amino schließt weiterhin gegebenenfalls substituiertes cyclisches Amino ein, wie gegebenenfalls substituiertes 5 oder 6-gliedriges cyclisches Amino, dass gegebenenfalls weitere Heteroatome wie beispielsweise N, O, S, bevorzugt O enthalten kann. Beispiele von solchen cyclischen Aminogruppen schließen ein: die vorstehend genannten stickstoffenthaltenden heterocyclischen Gruppen, die über ein Stickstoffatom angebunden sind, wie Piperidin-1-yl, 4-Hydroxy-piperidin-1-yl, 2-(Methoxycarbonyl)pyrrolidin-1-yl, Pyrrolidin-1-yl, Morpholin-4-yl, etc.

Gegebenenfalls substituiertes Aminocarbonyl leitet sich im Rahmen der Erfindung formal von gegebenenfalls substituiertem Amino, wie vorstehend erläutert, durch Hinzufügen eines Carbonylrestes ab ($(R)_2N-C(=O)-$). Dabei schließt gegebenenfalls substituiertes Amino erfindungsgemäß bevorzugt ein: Amino ($-NH_2$), gegebenenfalls substituiertes Mono- oder Dialkylamino (RHN-, $(R)_2N$-) wobei hinsichtlich der Definition von gegebenenfalls substituiertem Alkyl auf vorstehende Definition verwiesen werden kann. Ferner sind gegebenenfalls substituierte Mono- oder Diarylamino-Reste eingeschlossen oder gemischte gegebenenfalls substituierte Alkylarylamino-Reste, wobei hinsichtlich der Definition von gegebenenfalls substituiertem Alkyl oder Aryl auf vorstehende Definition verwiesen werden kann. Derartige Gruppen schließen beispielsweise Methylamino, Dimethylamino, Ethylamino, Hydroxyethylamino, wie 2-Hydroxyethylamino, Diethylamino, Phenylamino, Methylphenylamino etc. ein. Gegebenenfalls substituiertes Amino schließt weiterhin gegebenenfalls substituiertes cyclisches Amino ein, wie gegebenenfalls substituiertes 5 oder 6-gliedriges cyclisches Amino, dass gegebenenfalls weitere Heteroatome wie beispielsweise N, O, S, bevorzugt O enthalten kann. Beispiele von solchen cyclischen Aminogruppen schließen ein die vorstehend genannten stickstoffenthaltenden heterocyclischen Gruppen, die über ein Stickstoffatom angebunden sind, wie Piperidin-1-yl, 4-Hydroxy-piperidin-1-yl, 2-(Methoxycarbonyl)pyrrolidin-1-yl, Pyrrolidin-1-yl, Morpholin-4-yl, etc.

Beispiele von gegebenenfalls substituiertem Aminocarbonyl schließen demnach ein: Carbamoyl ($H_2NC(=O)-$), gegebenenfalls substituiertes Mono- oder Dialkylaminocarbonyl ($RHNC(=O)-$, $(R)_2NC(=O)-$) wobei hinsichtlich der Definition von gegebenenfalls substituiertem Alkyl auf vorstehende Definition verwiesen werden kann. Ferner sind gegebenenfalls substituierte Mono- oder Diarylaminocarbonyl-Reste eingeschlossen oder gemischte gegebenenfalls substituierte Alkylarylaminocarbonyl-Reste, wobei hinsichtlich der Definition von gegebenenfalls substituiertem Alkyl oder Aryl auf vorstehende Definition verwiesen werden kann. Bevorzugte substituierte Aminocarbonyl-Gruppen weisen bis zu 14 Kohlenstoffatome auf. Derartige Gruppen schließen beispielsweise Methylaminocarbonyl, Dimethylaminocarbonyl, Ethylaminocarbonyl, Diethylaminocarbonyl, Phenylaminocarbonyl, Diphenylaminocarbonyl, Methylphenylaminocarbonyl etc. ein.

In einer bevorzugten Ausführungsform bilden $R_1$ und $R_2$ oder $R_2$ und $R_3$ gemeinsam mit den Kohlenstoffatomen, an die sie gebunden sind, einen 5- oder 6-gliedrigen carbocyclischen Ring.

Bespiele der vorstehend erwähnten Ringbildung der Substituenten $R_1$ und $R_2$ oder $R_2$ und $R_3$ wie schematisch in den folgenden Formeln gezeigt:

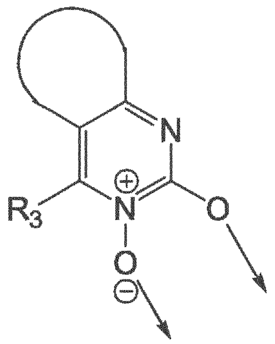

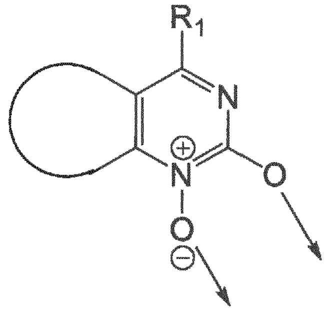

schließen insbesondere ein:

Verbindungen in denen $R_1$ und $R_2$ oder $R_2$ und $R_3$ bevorzugt zusammen eine Propylen ($-CH_2-CH_2-CH_2-$)- oder eine Butylen ($-CH_2-CH_2-CH_2-CH_2-$)-Gruppe darstellen, in denen gegebenenfalls jeweils eine Methylengruppe ($-CH_2-$) durch - O-, -NH-, oder $-NR_4-$ ersetzt sein kann, worin $R_4$ wie vorstehend erwähnt ist, und worin die von $R_1$ und $R_2$ oder $R_2$ und $R_3$ gebildeten Gruppen weiterhin gegebenenfalls jeweils durch einen bis drei Substituenten, ausgewählt aus der Gruppe, die besteht aus Hydroxy, Oxo, $C_{1-4}$-Alkoxy, Amino und Mono- oder Di-($C_{1-4}$-alkyl)amino, substituiert sein können.

Beispielhafte Liganden sind die folgenden:

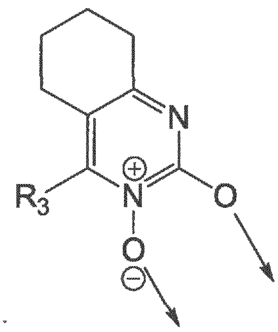

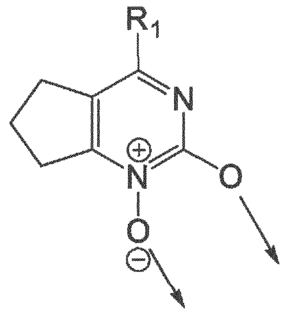

worin $R_1$ und $R_3$ jeweils wie vorstehend definiert sind.

In einer weiteren besonders bevorzugten Ausführungsform der erfindungsgemäßen Eisen(III)-Komplexverbindungen sind $R_1$, $R_2$, $R_3$ gleich oder verschieden und werden ausgewählt aus der Gruppe, die besteht aus Wasserstoff und Alkyl, mit der Maßgabe, dass mindestens einer, bevorzugt zwei der Substituenten $R_1$, $R_2$, und $R_3$ Alkyl sind. Alkyl ist dabei bevorzugt wie vorstehend erwähnt, insbesondere geradkettiges oder verzweigtes vorzugsweise unsubstituiertes Alkyl mit bis zu 6, bevorzugt bis zu 4 Kohlenstoffatomen. Noch bevorzugter sind Eisen(III)-Komplexverbindungen, worin $R_2$ Wasserstoff ist, und $R_1$ und $R_3$ jeweils gleich oder verschieden sind und aus Alkyl, wie vorstehend erwähnt, ausgewählt sind.

Besonders bevorzugt sind insbesondere die Eisen(III)-Komplexverbindungen der allgemeinen Formel (II):

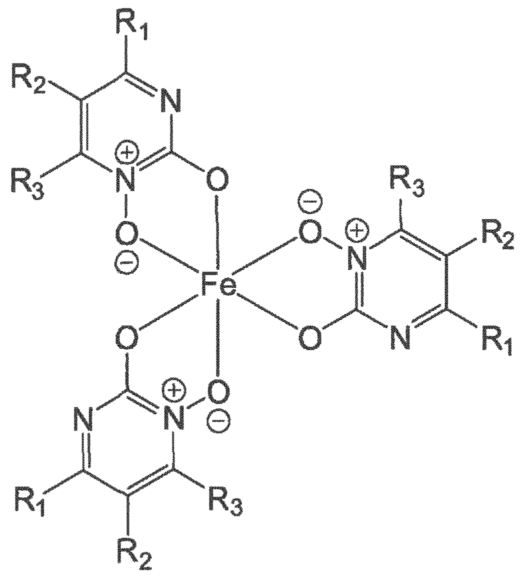

(II)

worin $R_1$, $R_2$, und $R_3$ jeweils wie oben oder bevorzugt wie nachstehend definiert sind.

Weiterhin bevorzugt sind $R_1$, $R_2$, und $R_3$ gleich oder verschieden und werden ausgewählt aus:

- Wasserstoff,
- $C_{1-6}$-Alkyl, bevorzugt wie vorstehend erläutert,
- Halogen, bevorzugt wie vorstehend erläutert,
- $C_{3-6}$-Cycloalkyl, bevorzugt wie vorstehend erläutert,
- $C_{3-6}$-Cycloalkyl-$C_{1-4}$-alkyl, bevorzugt wie vorstehend erläutert,
- $C_{1-4}$-Alkoxy-$C_{1-4}$-alkyl, bevorzugt wie vorstehend erläutert,
- $C_{1-4}$-Alkoxy-Carbonyl, bevorzugt wie vorstehend erläutert,
- $C_{1-4}$-Mono- oder Dialkylaminocarbonyl, bevorzugt wie vorstehend erläutert,
- Amino-Carbonyl bzw. Carbamoyl ($H_2NCO$-),
- Hydroxy-$C_{1-4}$-alkyl, bevorzugt wie vorstehend erläutert und
- Halogen-$C_{1-4}$-alkyl, bevorzugt wie vorstehend erläutert.

Besonders bevorzugt sind $R_1$, $R_2$, und $R_3$ gleich oder verschieden und werden ausgewählt aus: Wasserstoff, Halogen und $C_{1-6}$-Alkyl, bevorzugt wie vorstehend erläutert, insbesondere Wasserstoff, Chlor, Methyl, Ethyl und Propyl, insbesondere i-Propyl, Butyl, insbesondere sek.-Butyl. Am meisten bevorzugt werden $R_1$, $R_2$, und $R_3$ ausgewählt aus: Wasserstoff, Methyl und Ethyl. Erfindungsgemäß umfasst sind auch Eisen(III)-pyrimidin-2-ol 1-oxid-Komplexverbindungen in fester Form. Der Ausdruck "feste Form" meint hier insbesondere im Unterschied zur gelösten Form, bei dem die Eisen(III)-pyrimidin-2-ol 1-oxid-Komplexverbindungen gelöst in einem Lösungsmittel wie beispielsweise Wasser vorliegen. Der Ausdruck "in fester Form" meint des weiteren, dass die Eisen(III)-pyrimidin-2-ol 1-oxid-Komplexverbindungen bei Raumtemperatur (23°C) in fester Form vorliegen. Die Eisen(III)-pyrimidin-2-ol 1-oxid-Komplexverbindungen können dabei in amorpher, kristalliner oder teilkristalliner Form vorliegen. Auch können die Eisen(III)-pyrimidin-2-ol 1-oxid- Komplexverbindungen der Erfindung als Hydrate, insbesondere als kristalline Hydrate vorliegen, wie zum Beispiel als Monohydrat, insbesondere als kristallines Monohydrat.

Es ist für den Fachmann klar, dass die erfindungsgemäßen Liganden

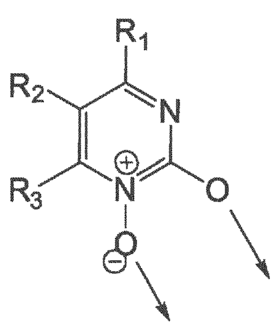

aus den entsprechenden Pyrimidin-2-ol-1-oxid-Verbindungen hervorgehen:

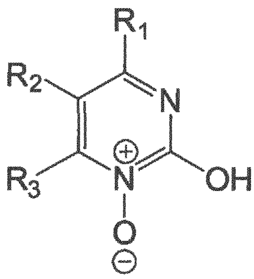

.

In den Pyrimidin-2-ol-1-oxid-Verbindungen liegt eine Keto-Enol-Tautomerie vor, bei der die Gleichgewichtslage durch verschiedene Faktoren bestimmt wird.

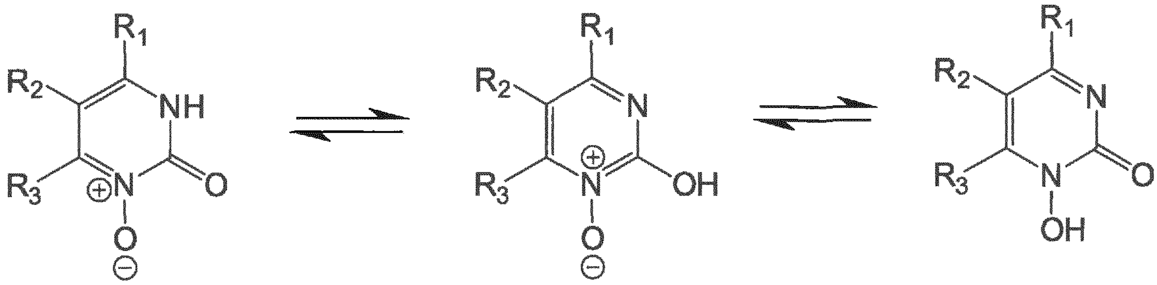

Der Ligand geht formal durch Abspalten eines Protons aus den entsprechenden Pyrimidin-1-oxid-verbindungen hervor:

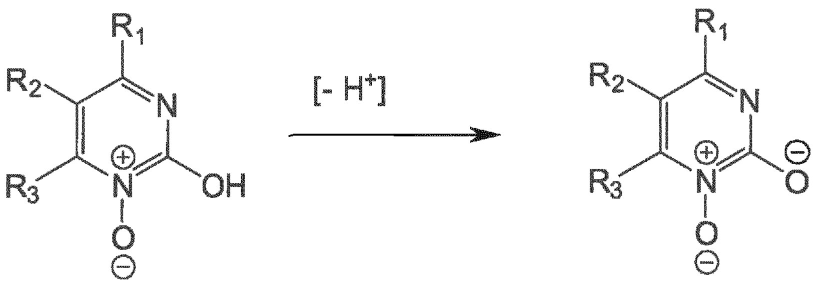


,

trägt also formal eine einfach negative Ladung.

Ausserdem ist es für den Fachmann klar, dass die erfindungsgemäßen verwendeten Pyrimidin-2-ol-1-oxid-Verbindungen in unterschiedlichen Schreibweisen (a, b und c) gezeichnet werden können, aber alle den gleichen Sachverhalt des N-Oxids beinhalten.

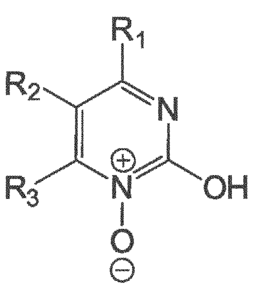

(a)

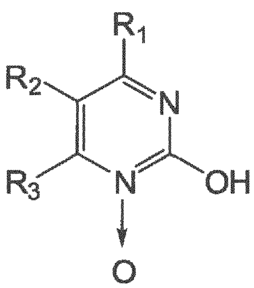

(b)

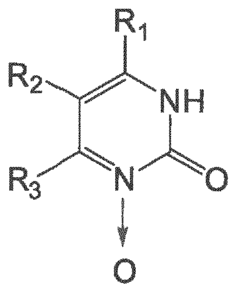

(c).

Die gilt entsprechend auch für die deprotonierte Form der Pyrimidin-2-ol-1-oxid-Liganden-Verbindungen. Im Rahmen der Erfindung sind sämtliche tautomere Formen enthalten auch wenn lediglich eine der mesomeren Grenzformeln gezeichnet ist.

Je nach Substituent $R_1$, $R_2$, und $R_3$ können auch diese an den tautomeren Grenzstrukturen im Pyrimidin-2-ol-1-oxid-Liganden teilhaben. Beispielhaft können hier die 4-Aminoverbindungen genannt werden. Zum Beispiel:

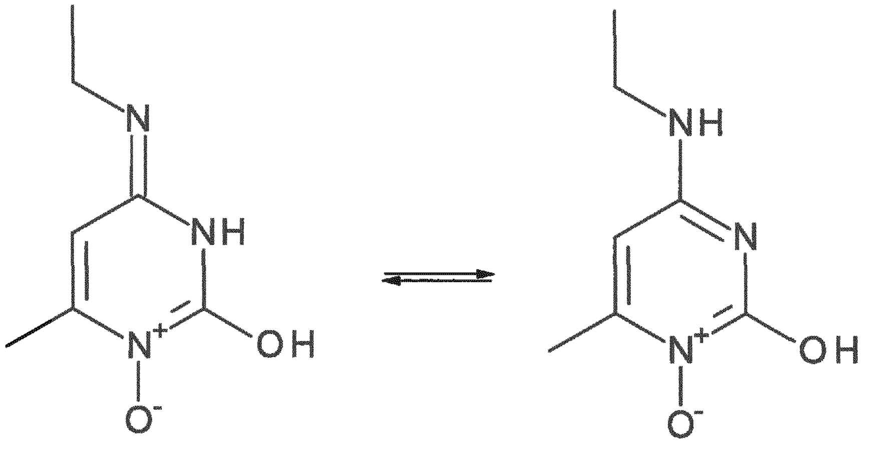

Sämtliche derartige Tautomere sind im Umfang der Erfindung enthalten.

Gegebenenfalls substituiertes Amino liegt vorzugsweise in der Position von $R_1$ vor, d.h, in der 4-Position der Pyrimidin-Liganden.

Die erfindungsgemäßen Eisen(III)-pyrimidin-2-ol-1-oxid-Komplexverbindungen insbesondere die der allgemeinen Formel (II) bzw. die entsprechenden Pyrimidin-2-ol-1-oxid-Liganden können in Form verschiedener Isomere oder Tautomere vorliegen. Isomere Formen schließen beispielsweise Regioisomere ein, die sich durch die Lage der Liganden zueinander unterscheiden, einschließlich sogenannter optischer Isomere, die sich wie Bild und Spiegelbild zueinander verhalten. Weiterhin können die Liganden bei Vorliegen asymmetrischer Kohlenstoffatome in Form optischer Isomere vorliegen, die sich wie Bild und Spiegelbild zueinander verhalten, und reine Enantiomere, Mischungen der Enantiomere, insbesondere Racemate umfassen. Enantiomerenreine Liganden können wie dem Fachmann bekannt ist durch optische Auflösungsverfahren, wie Umsetzung mit chiralen Reagenzien zu Diastereomeren, Trennung der Diastereomeren und Freisetzen der Enantiomere erhalten werden. Beispiele für tautomere Grenzstrukturen, welche erfindungsgemäß ebenfalls eingeschlossen sind, wurden vorstehend beispielhaft gezeigt.

Weitere bevorzugte Ausführungsformen der Erfindung schließen ein:

(In der vorliegenden Erfindung bedeuten die Ziffern 1-6 in "1-6C" oder "C1-6", bzw. "1-4" in "1-4C" oder "C1-4 "etc. jeweils die Anzahl der Kohlenstoffatome der sich daran anschließenden Kohlenwasserstoffreste-Bezeichnungen).

$R_1$ $R_2$ und $R_3$ wird ausgewählt aus der Gruppe, die besteht aus:

- Wasserstoff,
- Halogen,
- Mono- oder Di(1-6C-alkyl)amino,
- 1-6C-alkyl, (also Alkyl mit 1 bis 6 Kohlenstoffatomen),
- 3-6C-cycloalkyl,
- 3-6C-cycloalkyl-1-4C-atkyl,
- 1-4C-alkoxy-1-4C-alkyl,
- hydroxy-1-4C-alkyl,
- fluoro-1-4C-alkyl;

oder $R_1$ und $R_2$ bilden zusammen eine Propylen ($-CH_2-CH_2-CH_2$), Butylen ($-CH_2-CH_2-CH_2-CH_2-$), Azabutylen oder Oxabutylen- Gruppe;
oder $R_2$ und $R_3$ bilden zusammen eine Propylen ($-CH_2-CH_2-CH_2$), Butylen ($-CH_2-CH_2-CH_2-CH_2-$), Azabutylen oder Oxabutylen- Gruppe
oder $R_1$ und $R_2$ bilden zusammen mit den Kohlenstoffen an die sie gebunden sind, ein ungesättigten Ring, der gegebenenfalls ein oder zwei weitere Heteroatome aufweisen kann,
oder $R_2$ und $R_3$ bilden zusammen mit den Kohlenstoffen an die sie gebunden sind, ein ungesättigten Ring, der gegebenenfalls ein oder zwei weitere Heteroatome aufweisen kann,

oder pharmazeutisch verträgliche Salze davon.

Besonders bevorzugt werden die vorstehend genannten Substituentengruppen wie folgt definiert:

1-6C-Alkyl beinhaltet bevorzugt geradkettige oder verzweigte Alkylgruppen mit 1 bis 6 Kohlenstoffatomen. Beispiele hierfür können Methyl, Ethyl, n-Propyl, isoPropyl, n-Butyl, iso-Butyl, sec-Butyl, tert-Butyl n-Pentyl, iso-Pentyl, neo-Pentyl, n-Hexyl, iso-Hexyl und neo-Hexyl sein.

3-6C-Cycloalkyl beinhaltet bevorzugt Cycloalkyl mit 1 bis 6 Kohlenstoffatomen, wie Cyclopropyl, Cyclobutyl, Cyclopentyl, Cyclohexyl.

3-6C-Cycloalkyl-1-4C-alkyl beinhaltet bevorzugt eine oben beschriebene 1-6C-Alkyl Gruppe, substituiert mit einer oben beschriebenen 3-6C-Cycloalkyl Gruppe. Beispiele hierfür können eine Cyclopropylmethyl, Cyclopentylmethyl und Cyclohexylmethyl Gruppe sein.

**1-3C-alkoxy-carbonyl-1-6C-alkyl,** beinhaltet bevorzugt eine oben beschriebene 1-6C-Alkyl Gruppe, die mit einer Carbonylgruppe, die mit einer 1-3C Alkoxy Gruppe als Carbonsäureester vorliegt, verknüpft ist. Beispiele hierfür können ein Methoxycarbonylmethyl, Ethoxycarbonylmethyl, Methoxycarbonylethyl, Ethoxycarbonylethyl und Isopropoxycarbonylmethyl.

**1-4C-Alkoxy** beinhaltet bevorzugt eine 1-4C-Alkoxy Gruppe, bei der ein Sauerstoffatom mit einer geraden oder verzweigten Alkylkette mit 1-4 Kohlenstoffatome verbunden ist. Beispiele dieser Gruppe können Methoxy, Ethoxy, Propoxy und Isobutoxy sein.

**1-4C-Alkoxy-1-4C-alkyl** beinhaltet bevorzugt eine oben beschriebene 1-4C-Alkoxy Gruppe, die mit einer oben beschriebenen 1-4C-alkyl Gruppe verknüpft ist. Beispiele dieser Gruppe können Methoxyethyl, Ethoxypropyl, Methoxypropyl, Isobutoxymethyl sein.

**Hydroxy-1-4C-alkyl** beinhaltet eine oben beschriebene 1-4C-Alkyl Gruppe, die mit einer Hydroxygruppe substituiert ist. Beispiele hier können Hydroxyethyl, Hydroxybutyl und Hydroxyisopropyl sein.

**Fluor-1-4C-alkyl** beinhaltet eine oben beschriebene 1-4C-Alkyl Gruppe, die mit eins bis drei Fluoratomen substituiert ist. Beispiele hier können Trifluormethyl und Trifluorethyl sein.

**Halogen** bedeutet F, Cl, Br, I.

Besonders bevorzugt wird:

$\mathbf{R_1}$ $\mathbf{R_2}$ und $\mathbf{R_3}$ ausgewählt aus der Gruppe, die besteht aus:

- Wasserstoff,
- Halogen,
- 1-6C-alkyl,
- 1-4C-alkoxy-1-4C-alkyl,
- hydroxy-1-4C-alkyl;

oder $\mathbf{R_1}$ und $\mathbf{R_2}$ bilden zusammen eine Propylen ($-CH_2-CH_2-CH_2$), Butylen ($-CH_2-CH_2-CH_2-CH_2-$), Azabutylen oder Oxabutylen- Gruppe;
oder $\mathbf{R_2}$ und $\mathbf{R_3}$ bilden zusammen eine Propylen ($-CH_2-CH_2-CH_2$), Butylen ($-CH_2-CH_2-CH_2-CH_2-$), Azabutylen oder Oxabutylen- Gruppe,
oder $R_1$ und $R_2$ bilden zusammen mit den Kohlenstoffen an die sie gebunden sind, ein ungesättigten Ring, der ein weiteres Heteroatom aufweisen kann,
oder $R_2$ und $R_3$ bilden zusammen mit den Kohlenstoffen an die sie gebunden sind, ein ungesättigten Ring, der ein weiteres Heteroatom aufweisen kann.

Ganz besonders bevorzugt wird:

$\mathbf{R_1}$, $\mathbf{R_2}$ und $\mathbf{R_3}$ ausgewählt aus der Gruppe, die besteht aus:

- Wasserstoff

- 1-6C-alkyl;
- 1-4C-alkoxy-1-4C-alkyl

oder **$R_1$** und **$R_2$** bilden zusammen eine Propylen ($-CH_2-CH_2-CH_2$) oder Butylen ($-CH_2-CH_2-CH_2-CH_2-$) Gruppe; oder **$R_2$** und **$R_3$** bilden zusammen eine Propylen ($-CH_2-CH_2-CH_2$) oder Butylen ($-CH_2-CH_2-CH_2-CH_2-$) - Gruppe oder $R_1$ und $R_2$ bilden zusammen mit den Kohlenstoffen an die sie gebunden sind, ein ungesättigten Ring, der ein weiteres Stickstoffatom aufweisen kann,
oder $R_2$ und $R_3$ bilden zusammen mit den Kohlenstoffen an die sie gebunden sind, ein ungesättigten Ring, der ein weiteres Stickstoffatom aufweisen kann.

Besonders bevorzugte Komplex-Verbindungen der allgemeinen Formel (II) sind in den Beispielen beschrieben.

Die Erfindung betrifft weiterhin ein Verfahren zur Herstellung der erfindungsgemäßen Eisen(III)-Komplexverbindungen, welches die Umsetzung eines Pyrimidin-2-ol-1-oxids der Formel (III) mit einem Eisen(III)-salz umfasst.

Pyrimidin-2-ol-1-oxide als Ausgangsverbindungen schließen dabei insbesondere diejenigen der Formel (III) ein:

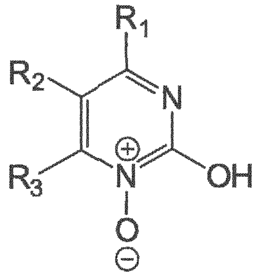

(III)

worin $R_1$, $R_2$ und $R_3$ wie oben definiert sind, wobei auf die tautomeren Grenzstrukturen hingewiesen wurde.

Beispiele geeigneter Eisen(III)-salze schließen ein: Eisen(III)-chlorid, Eisen(III)-acetat, Eisen(III)-sulfat, Eisen(III)-nitrat und Eisen(III)-acetylacetonat, worunter Eisen(III)-chlorid bevorzugt ist.

Ein bevorzugtes Verfahren ist im folgenden Schema gezeigt:

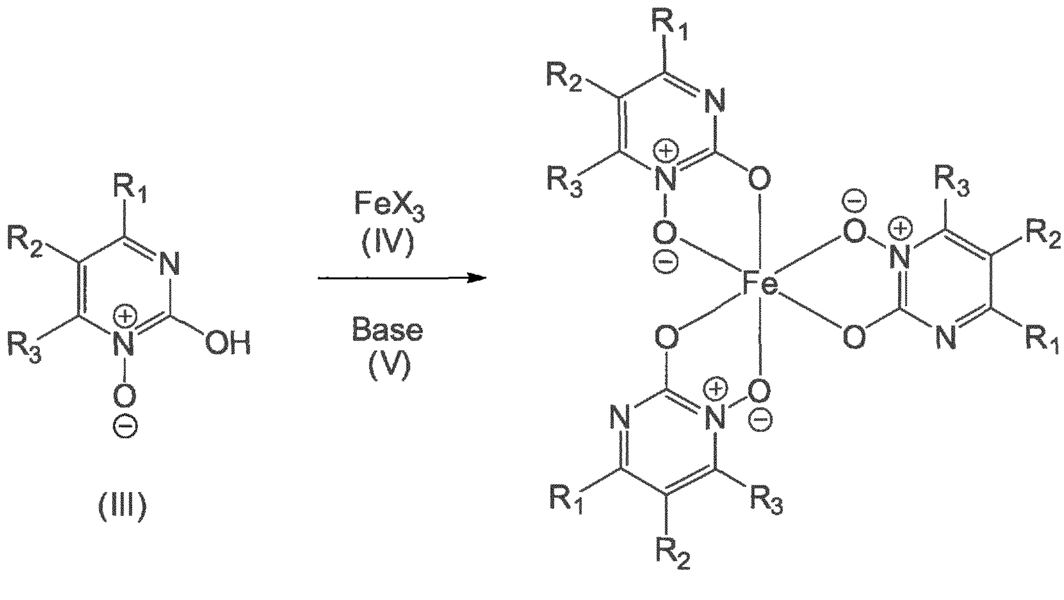


(III) (II)

worin $R_1$, $R_2$ und $R_3$ wie oben definiert sind, X ein Anion wie Halogenid, wie Chlorid, ein Carboxylat, wie Acetat, Sulfat, Nitrat und Acetylacetonat ist und Base eine übliche organische oder anorganische Base darstellt.

Beim erfindungsgemäßen Verfahren werden bevorzugt 3 eq der Pyrimidin-2-ol-1-oxide (III) unter Verwendung von geeigneten Eisen(III)-salzen (IV) (besonders eignen sich hier Fe(III)-chlorid, Fe(III)-acetat, Fe(III)-sulfat und Fe(III)-acetylacetonat) zu den entsprechenden Komplexen der allgemeinen Formel (II) unter Standardbedingungen umgesetzt. Hierbei wird die Synthese unter den für die Komplexbildung optimalen pH-Bedingungen durchgeführt. Der

optimale pH-Wert wird durch Zugabe von Base (V), besonders eignet sich hier die Verwendung von Natrium-Hydroxyd, Natrium-Carbonat, NatriumHydrogencarbonat, Natrium-Methanolat, Kalium-Hydroxyd Kaliumcarbonat, Kalium-Hydrogencarbonat oder Kalium-Methanolat, eingestellt.

Die für die Darstellung der Komplexe benötigten Liganden (III) wurden nach folgender Synthesemethode (analog Tetrahedron 1967, 23, 353-357) durchgeführt. Hierzu wurden die käuflichen oder synthetisierten 1-3-Dicarbonylverbindungen der allgemeinen Formel (IV) mit Hydroxyharnstoff (V) unter Standardbedingungen zum Liganden der allgemeinen Formel (III) umgesetzt. Dabei kommt es bei dieser Synthese bei Verwendung unsymmetrischer 1-3-Dicarbonylverbindungen fast immer zum Auftreten des entsprechenden Regioisomers (IIIa), das unter dem Fachmann vertrauten Standardmethoden abgetrennt werden kann. Bei bestimmten Substitutionsmustern für $R_1$, $R_2$ und $R_3$ kann (IIIa) auch das Hauptprodukt darstellen und ist in diesen Fällen dann der Synthesezugang für diese entsprechenden Substitutionsmuster.

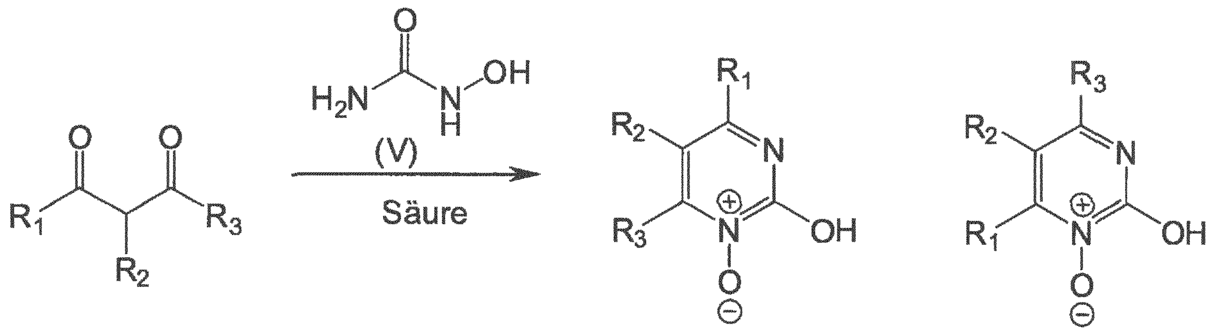


(IV) (III) (IIIa)

Analog hierzu können auch leicht modifizierte Syntheserouten zur Darstellung der entsprechenden Liganden der allgemeinen Formel (III) verwendet werden. So wird bei der Synthese von Ohkanda et.al (Bull. Chem. Soc. Jpn. 1993, 66, 841-847) der benzylgeschützte Harnstoff der allgemeinen Formel (V-Bn) unter Standardbedingungen mit den entsprechenden 1-3-Dicarbonylverbindungen (IV) zum entsprechenden benzylgeschützten Produkt (III-Bn) cyclisiert, wobei dann die anschliessende Abspaltung zum gewünschten Produkt (III) führt. Auch bei dieser alternativen Syntheseroute kommt es zum Auftreten von (IIIa).

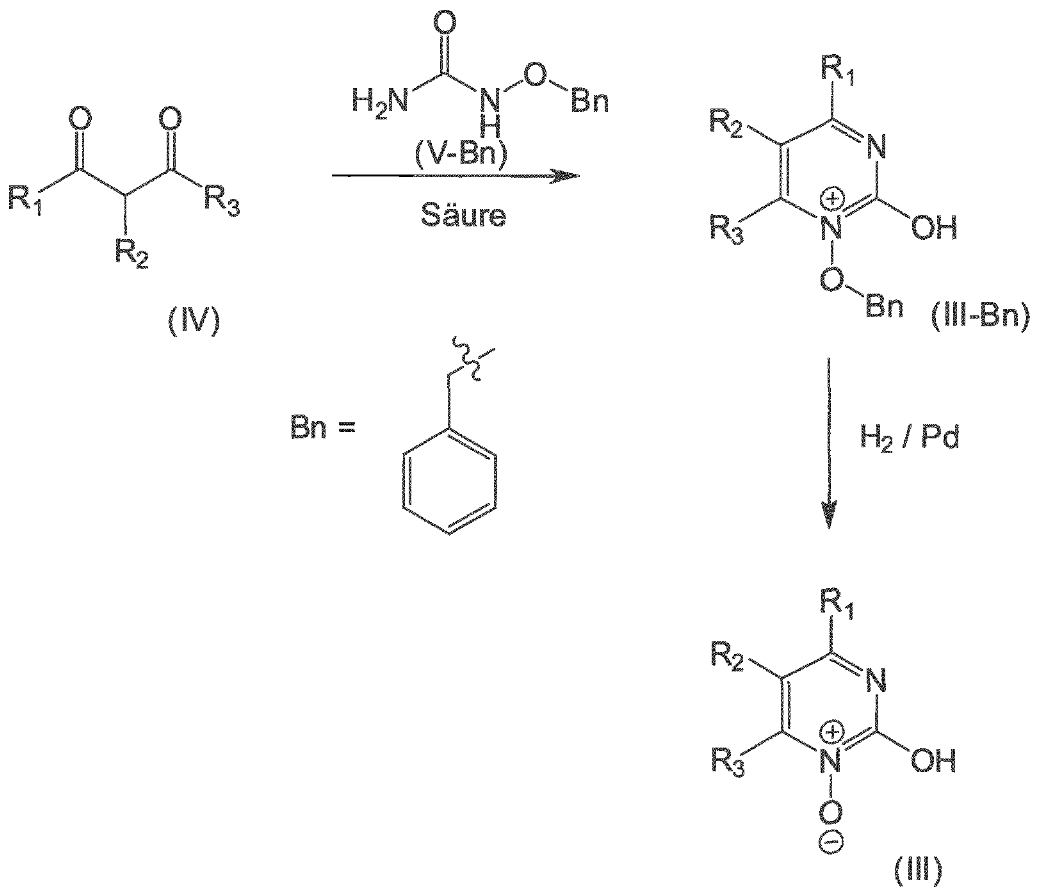


(IV) (V-Bn) (III-Bn) (III)

Für Liganden, bei denen einer oder beide der Reste $R_1$ und $R_3$ Wasserstoff entspricht, wurde eine leicht modifizierte Synthese durchgeführt. Hierbei wurden die entsprechenden geschützten 1,3-Dicarbonylverbindungen, wie zum Beispiel diejenigen der allgemeinen Formel (VI) analog mit Hydroxyharnstoff (V) unter sauren Standardbedingungen umgesetzt. Wobei hier R vorzugsweise Methyl oder Ethyl ist.

Auch hierbei wird das Produktverhältnis der beiden sich bildenden Pyrimidin-2-ol-1-oxide (IIIc) und (IIId) durch die Wahl der Reste $R_1$ und $R_2$ gesteuert. Die Auftrennung erfolgt dann unter dem Fachmann vertrauten Standardbedingungen.

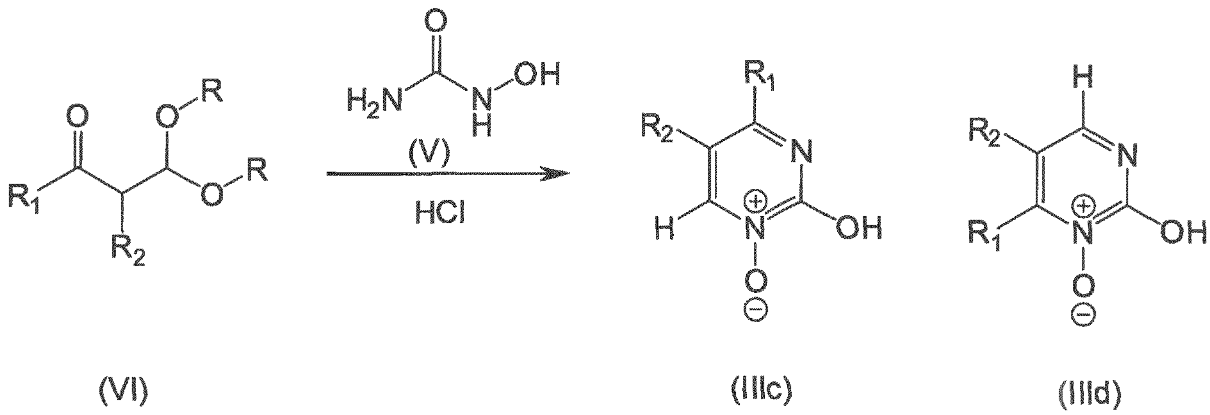


(V)

(VI) (IIIc) (IIId)

Allgemein lässt sich die Darstellung der Pyrimidin-2-ol-1-oxide (III) auch durch andere, dem Fachmann vertrauten, Syntheserouten durchführen. Somit besteht beispielsweise die Möglichkeit, die entsprechend substituierten Pyrimidine (VII) durch Umsetzung mit geeigneten Oxidationsmitteln, wie Wasserstoffperoxid oder Peroxycarbonsäuren zu den gewünschten Produkten der allgemeinen Formel (III) darzustellen (z. B. analog Can. J. Chem. 1984, 62, 1176-1180).

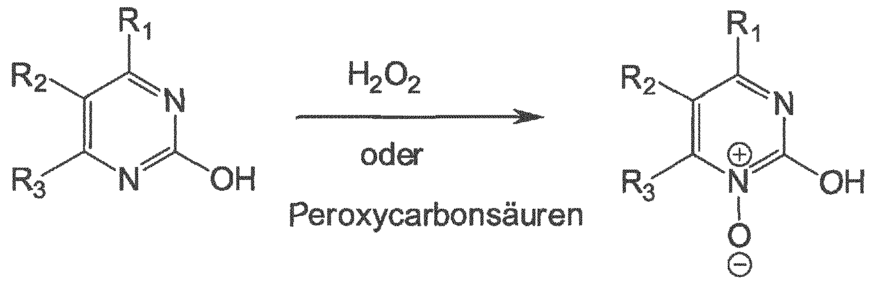


(VII) (III)

Beispiele der Pyrimidin-2-ol-1-oxid-Ausgangsverbindungen (III) schließen insbesondere die folgenden ein:

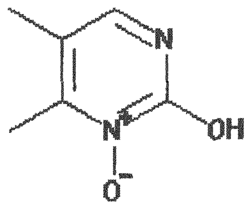

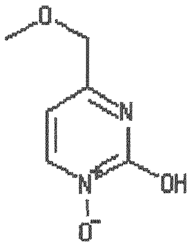

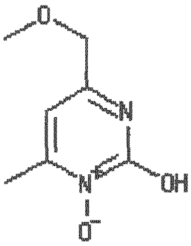

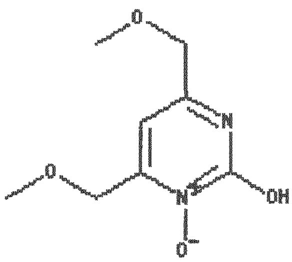

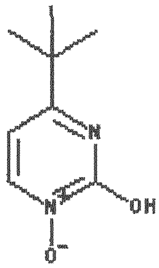

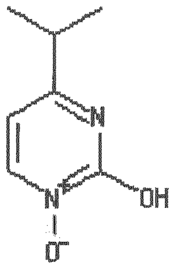

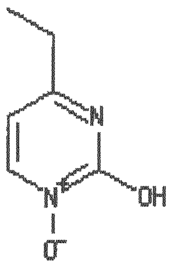

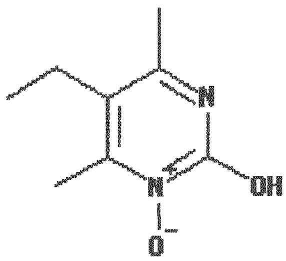

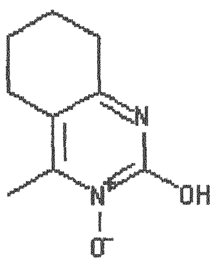

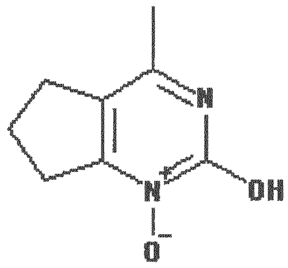

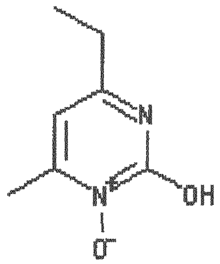

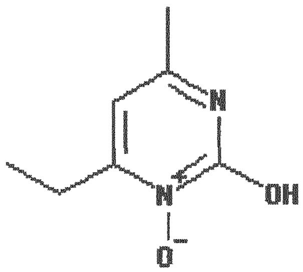

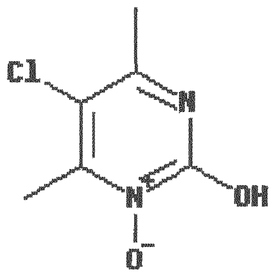

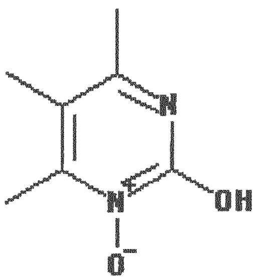

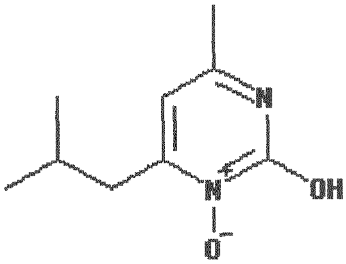

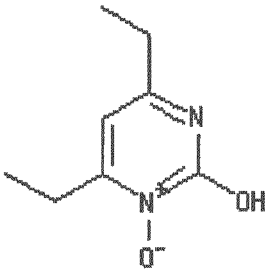

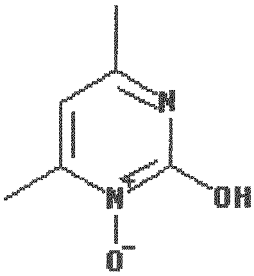

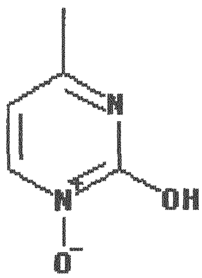

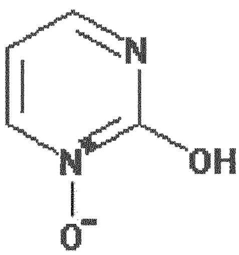

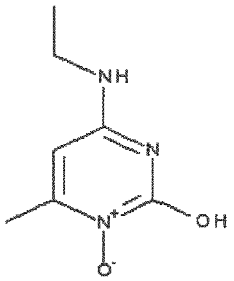

.

Aus diesen Verbindungen gehen die Liganden der erfindungsgemäßen Eisen-Komplexverbindungen durch einfache Deprotonierung an der Hydroxygruppe hervor.

Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate, Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.

Pharmazeutisch annehmbaren Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxylmethyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-pyrimidin-2-ol 1-oxid-Komplexverbindungen, die Gegenstand der vorliegenden Erfindung sind, und die insbesondere durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder depressiven Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3 -5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien

bei inflammatorischen Darmerkrankungen (IBD; inflammatory bowel diseases).

Die Verabreichung kann über einen Zeitraum von mehreren Monaten bis zur Verbesserung des Eisenstatus, reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des Gesundheitszustandes erfolgen.

Das erfindungsgemäße Präparat kann von Kindern, Jugendlichen und Erwachsenen eingenommen werden.

Die erfindungsgemäßen Verbindungen angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindungen sowie gegebenenfalls einen oder mehrere pharmakologisch verträgliche Träger und/oder Hilfsstoffe und/oder Lösungsmittel. Die genannten pharmazeutische Zusammensetzungen enthalten beispielsweise bis 99 Gew.-% oder bis zu 90 Gew.-% oder bis zu 80 Gew.-% der erfindungsgemäßen Verbindungen, wobei der Rest jeweils durch pharmakologisch verträgliche Träger und/oder Hilfsstoffe und/oder Lösungsmittel gebildet wird,

Dabei handelt es sich um übliche pharmazeutische Träger, Hilfsstoffe oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistenten Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

Bevorzugt werden die erfindungsgemäßen Verbindungen sowie pharmazeutische Zusammensetzungen, enthaltend solche Verbindungen oral appliziert, obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistenten Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzungen verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat),

ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssige Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## EXPERIMENTELLER TEIL

Die Ligandbezeichnungen wurden nach IUPAC-Nomenklatur mit dem Programm ACD/Name, Version 12.01 von Advanced Chemistry Development Inc. erstellt.

Abkürzungen

| | | | |
|---|---|---|---|
| s | Singulett | t | Triplett |
| d | Duplett | q | Quartett |
| dd | Doppel-Duplett | m | Multiplett (breit/überlagert) |
| L | Ligand | | |

### Ausgangsverbindungen:

**A. Pyrimidin-2-ol 1-oxid Hydrochlorid**

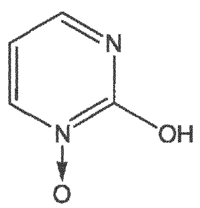

0.261 mol (19.85 g) Hydroxyharnstoff wurden in 390 ml 1 M HCl gelöst und unter Eiskühlung 0.235 mol (51.77 g) 1,1,3,3-Tetraethoxypropan zugetropft, wobei die Innentemperatur auf 1-2°C gehalten wurde. Die Lösung wurde im Eisbad auf Raumtemperatur aufgetaut und über Nacht gerührt, dann zur Trockne eingeengt. Der Rückstand wurde mit 250 ml Aceton aufgeschlämmt, das Gemisch wurde im Eis/Ethanolbad gekühlt, der Feststoff abfiltriert und mit wenig eiskaltem Aceton nachgewaschen. Nach dem Trocknen wurden 24.5 g Rohprodukt erhalten. Das Rohprodukt wurde

mit 460 ml Methanol aus der Siedehitze umkristallisiert, im Eis/Ethanolbad gekühlt, abfiltriert und getrocknet. Die Mutterlauge wurde am Rotationsverdampfer eingeengt, bis erneut Produkt auszufallen begann, dann wurde eine zweite Fraktion analog kristallisiert. Nach dem Trocknen wurden 11.8 g (1. Fraktion) und 5.9 g (2. Fraktion) der Titelverbindung erhalten.
IR (in Substanz, $cm^{-1}$): 3114, 3082, 2995, 2935, 2837, 2776, 2718, 2467, 1734, 1575, 1492, 1421, 1363, 1314, 1232, 1176, 1123, 1100, 1073, 911, 861, 773, 733, 689, 574, 521 (2. Fraktion).
CN-Elementaranalyse: C, 32.29; N, 18.98 (1. Fraktion); C, 32.41; N, 18.98 (2. Fraktion).
Chlorid-Gehalt: 24.6% (m/m) (1. Fraktion), 23.6% (m/m) (2. Fraktion)
LC-MS: 113 (M+H).
1 H-NMR (DMSO-$d_6$, 400 MHz): $\delta$ [ppm] = 9.05 (dd, 1 H), 8.55 (dd, 1 H), 6.74 (t, 1 H).

**B. 4-Methylpyrimidin-2-ol 1-oxid Hydrochlorid**

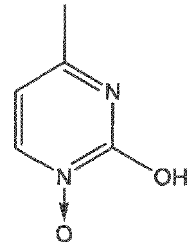

0.10 mol (7.61 g) Hydroxyharnstoff wurden in 150 ml 1 M HCl gelöst und unter Eiskühlung 0.11 mol (16.15 g, techn. 90%) 4,4-Dimethoxy-2-butanon zugetropft, wobei die Innentemperatur auf 1-3°C gehalten wurde. Die Lösung wurde im Eisbad auf Raumtemperatur aufgetaut und über Nacht gerührt, dann filtriert und zur Trockne eingeengt. Der Rückstand wurde mit 100 ml Aceton aufgeschlämmt, das Gemisch wurde auf -18°C gekühlt, der Feststoff abfiltriert und mit wenig eiskaltem Aceton nachgewaschen. Nach dem Trocknen wurden 10.7 g Rohprodukt erhalten. Das Rohprodukt wurde mit 1750 ml Ethanol aus der Siedehitze umkristallisiert, im Eis/Ethanolbad gekühlt, abfiltriert, mit wenig Ethanol nachgewaschen und getrocknet (Fraktion 1). Die Mutterlauge wurde am Rotationsverdampfer eingeengt, bis erneut Produkt auszufallen begann, kühl gestellt und eine zweite Fraktion isoliert. Nach dem Trocknen wurden 6.15 g (1. Fraktion) und 1.86 g (2. Fraktion) Produkt erhalten. Beide Fraktionen wurden vereint und mit 230 ml 90% Ethanol (10% Wasser) aus der Siedehitze analog umkristallisiert. Nach dem Trocknen wurden 3.75 g (3. Fraktion) und 2.36 g (4. Fraktion) der Titelverbindung erhalten.
IR (in Substanz, $cm^{-1}$): 3102, 3034, 2926, 2841, 2730, 2657, 2531, 1741, 1650, 1602, 1581, 1518, 1456, 1374, 1302, 1184, 1131, 1110, 1037, 977, 888, 821, 780, 734, 697, 603 (3.Fraktion).
CHN-Elementaranalyse: C, 37.48; H, 4.33; N, 17.12 (3.Fraktion); C, 37.12; H, 4.30; N, 17.08 (4.Fraktion). LC-MS: 127 (M+H).
1 H-NMR (DMSO-$d_6$, 400 MHz): $\delta$ [ppm] = 8.97 (d, 1 H), 6.73 (d, 1 H), 2.49 (s, 3H).

**C. 4,6-Dimethylpyrimidin-2-ol 1-oxid Hydrochlorid**

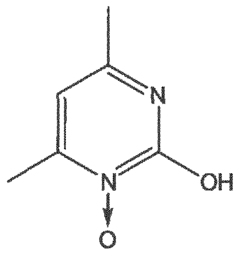

0.40 mol (30.6 g) Hydroxyharnstoff wurden in 600 ml 1 M HCl gelöst und unter Eiskühlung 0.50 mol (50.06 g) Acetylaceton zugetropft, wobei die Innentemperatur auf 1-3°C gehalten wurde. Die Lösung wurde im Eisbad auf Raumtemperatur aufgetaut und über Nacht gerührt, dann filtriert und zur Trockne eingeengt. Der Rückstand wurde mit 400

ml Aceton aufgeschlämmt, das Gemisch wurde auf -18°C gekühlt, der Feststoff abfiltriert und mit wenig eiskaltem Aceton nachgewaschen. Nach dem Trocknen wurden 45.5 g Rohprodukt erhalten. Das Rohprodukt wurde mit 4.0 l Ethanol aus der Siedehitze umkristallisiert, auf -18°C gekühlt, abfiltriert, mit wenig Ethanol nachgewaschen und getrocknet (Fraktion 1). Die Mutterlauge wurde am Rotationsverdampfer eingeengt, bis erneut Produkt auszufallen begann, kühl gestellt und eine zweite Fraktion isoliert. Nach dem Trocknen wurden 26.0 g (1. Fraktion, Reinheit >98%) und 11.0 g (2. Fraktion, enthielt 32% $NH_4Cl$ als Nebenprodukt) der Titelverbindung erhalten.
IR (in Substanz, $cm^{-1}$): 3077, 3052, 2938, 2855, 2796, 2521, 1740, 1610, 1564, 1509, 1423, 1368, 1316, 1215, 1163, 1141, 1089, 1049, 1026, 997, 975, 852, 775, 741, 695, 626, 601 (1. Fraktion). CHN-Elementaranalyse: C, 40.74; H, 5.03; N, 15.78 (1. Fraktion); C, 27.75; H, 5.87; N, 18.98 (2. Fraktion).
Chlorid-Gehalt: 19.9% (m/m) (1. Fraktion), 33.4% (m/m) (2. Fraktion)
LC-MS: 141 (M+H).
1 H-NMR (DMSO-$d_6$, 400 MHz): $\delta$ [ppm] = 6.78 (s, 1 H); 2.53 (s, 3H); 2.43 (s, 3H) (1. Fraktion).

**D. 4,6-Diethylpyrimidin-2-ol 1-oxid Hydrochlorid**

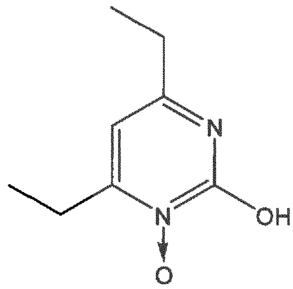

0.19 mol (14.45 g) Hydroxyharnstoff wurden in 300 ml 1 M HCl gelöst, 300 ml Methanol zugegeben und unter Eiskühlung 0.19 mol (24.35 g) 3,5-Heptandion zugetropft, wobei die Innentemperatur auf 1-2°C gehalten wurde. Die Lösung wurde im Eisbad auf Raumtemperatur aufgetaut und über Nacht gerührt, dann zur Trockne eingeengt. Der Rückstand wurde mit 200 ml Aceton aufgeschlämmt, das Gemisch im Eis/Ethanolbad auf unter 0°C gekühlt, der Feststoff abfiltriert und mit wenig eiskaltem Aceton nachgewaschen. Nach dem Trocknen wurden 7.88 g eines Produkts erhalten, das zu 48% die Titelverbindung und zu 52% das Nebenprodukt Ammoniumchlorid enthielt.
IR (in Substanz, $cm^{-1}$): 3116, 3024, 2804, 2687, 2628, 1996, 1746, 1603, 1572, 1512, 1443, 1394, 1297, 1213, 1156, 1082, 1061, 963, 901, 861, 814, 745, 700.
CHN-Elementaranalyse: C, 22.71; H, 6.94; N, 19.99.
Chlorid-Gehalt: 42.9% (m/m)
LC-MS: 169 (M+H).
1 H-NMR (DMSO-$d_6$, 400 MHz): $\delta$ [ppm] = 6.75 (s, 1 H), 2.86 (q, 2H), 2.70 (q, 2H), 1.21 (t, 3H), 1.20 (t, 3H).

**E. 4-Methyl-6-(2-methylpropyl)pyrimidin-2-ol 1-oxid Hydrochlorid**

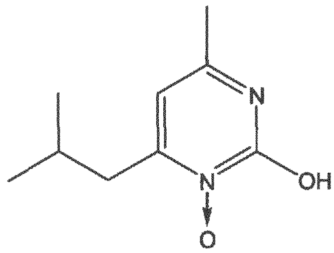

0.20 mol (15.21 g) Hydroxyharnstoff wurden in 300 ml 1 M HCl gelöst, 300 ml Methanol zugegeben und unter Kühlung bei -12 bis -10°C 0.20 mol (28.44 g) 6-Methyl-2,4-heptandion zugetropft. Die Lösung wurde langsam auf Raumtemperatur erwärmen lassen und über Nacht gerührt, dann zur Trockne eingeengt. Der Rückstand wurde mit 200

ml Aceton aufgeschlämmt, das Gemisch im Eis/Ethanolbad auf unter 0°C gekühlt, der Feststoff abfiltriert und mit wenig eiskaltem Aceton nachgewaschen. Nach dem Trocknen wurden 7.23 g eines Produkts erhalten, das zu 58% die Titelverbindung und zu 42% das Nebenprodukt Ammoniumchlorid enthielt.

IR (in Substanz, $cm^{-1}$): 3106, 3011, 2963, 2576, 1834, 1740, 1604, 1567, 1514, 1467, 1446, 1403, 1371, 1321, 1280, 1234, 1209, 1149, 1104, 1070, 1032, 1010, 915, 861, 820, 774, 750, 712, 680, 644, 615, 580.

CHN-Elementaranalyse: C, 28.68; H, 6.93; N, 18.33.

Chlorid-Gehalt: 37.6% (m/m)

LC-MS: 183 (M+H).

1 H-NMR (DMSO-$d_6$, 400 MHz): $\delta$ [ppm] = 6.76 (s, 1H), 2.72 (d, 2H), 2.44 (s, 3H), 2.13 (m, 1 H), 0.91 (d, 6H). Aus dem NMR-Spektrum wurde abgeschätzt, dass das Produkt ca. 2% des Regioisomeren 6-Methyl-4-(2-methylpropyl)pyrimidin-2-ol 1-oxid Hydrochlorid enthielt.

**F. 4,5,6-Trimethylpyrimidin-2-ol 1-oxid Hydrochlorid**

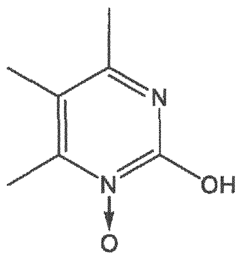

0.263 mol (20.0 g) Hydroxyharnstoff wurden in 263 ml 1 M HCl gelöst und unter Eiskühlung 0.876 mol (100 g) 3-Methyl-2,4-pentandion (95%, Alfa Aesar) zugetropft. Das Zweiphasengemisch wurde 1 h bei Raumtemperatur gerührt, dann wurde es zweimal mit je 530 ml Ethylacetat extrahiert. Die vereinigten organischen Phasen wurden mit Natriumsulfat getrocknet und am Rotationsverdampfer zur Trockne eingeengt. Es wurden 86.6 g von mit Acetylaceton abgereichertem 3-Methyl-2,4-pentandion erhalten.

0.758 mol (60.84 g) Hydroxyharnstoff wurden in 500 ml 2 M HCl gelöst und 200 ml Methanol sowie 0.758 mol (86.56 g) 3-Methyl-2,4-pentandion zugegeben. Die Lösung wurde 1 h bei 50°C gerührt, dann zur Trockne eingeengt. Der Rückstand wurde in 80 ml Aceton suspendiert, das Gemisch im Eis/Ethanolbad auf unter 0°C gekühlt, der Feststoff abfiltriert, mit wenig eiskaltem Aceton nachgewaschen und getrocknet. 21.7 g Zwischenprodukt wurden in 150 ml Methanol zum Sieden erhitzt, heiss von unlöslichen Anteilen abfiltriert und erneut zur Trockne eingedampft. Es wurden 7.05 g Feststoff (1. Fraktion) erhalten, das zu 9.6% die Titelverbindung und zu 90% das Nebenprodukt Ammoniumchlorid enthielt.

Aus der Aceton-Mutterlauge fiel weiterer Feststoff aus, der abfiltriert, mit wenig Aceton nachgewaschen und getrocknet wurde. 2.50 g des Feststoffs (2. Fraktion) enthielten zu 82% die Titelverbindung und zu 18% das Nebenprodukt Ammoniumchlorid.

IR (in Substanz, $cm^{-1}$): 3112, 2997, 2934, 2850, 2796, 2629, 2544, 1734, 1612, 1582, 1513, 1472, 1392, 1373, 1309, 1247, 1215, 1152, 1132, 1093, 1014, 945, 896,803,777,742,707,630,603,558,528,500.

CHN-Elementaranalyse: C, 36.31; H, 6.16; N, 16.28 (2. Fraktion).

Chlorid-Gehalt: 25.9% (m/m) (2. Fraktion) LC-MS: 155 (M+H).

1 H-NMR (DMSO-$d_6$, 400 MHz): $\delta$ [ppm] = 2.56 (s, 3H), 2.47 (s, 3H), 2.05 (s, 3H). Aus dem NMR-Spektrum wurde abgeschätzt, dass das Produkt ca. 9% 4,6-Dimethylpyrimidin-2-ol 1-oxid Hydrochlorid als Nebenprodukt enthielt.

**G. 5-Chloro-4,6-dimethylpyrimidin-2-ol 1-oxid Hydrochlorid**

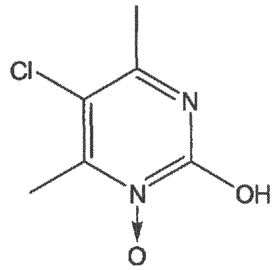

0.20 mol (15.21 g) Hydroxyharnstoff wurden in 300 ml 1 M HCl gelöst, 300 ml Methanol zugegeben und unter Eiskühlung bei 1-2°C 0.20 mol (26.91 g) 3-Chlor-2,4-pentandion zugetropft. Das Zweiphasengemisch wurde langsam auf Raumtemperatur erwärmen lassen und über Nacht gerührt. Die dann klare Lösung wurde zur Trockne eingeengt. Der Rückstand wurde mit 200 ml Aceton aufgeschlämmt, das Gemisch im Eis/Ethanolbad auf unter 0°C gekühlt, der Feststoff abfiltriert und mit wenig eiskaltem Aceton nachgewaschen. Das Filtrat wurde zur Trockne eingeengt, der Rückstand in 20 ml Tetrahydrofuran suspendiert, abfiltriert, mit wenig Tetrahydrofuran gewaschen und getrocknet. Es wurden 1.40 g eines Produkts erhalten, das zu 53% die Titelverbindung und zu 47% das Nebenprodukt Ammoniumchlorid enthielt.

IR (in Substanz, $cm^{-1}$): 3115, 2900, 2667, 2516, 1745, 1577, 1505, 1378, 1310, 1194, 1135, 1102, 1049, 973, 900, 835, 737, 674, 578.

Chlorid-Gehalt: 40.1% (m/m)

LC-MS: 175 (M+H).

1 H-NMR (DMSO-$d_6$, 400 MHz): $\delta$ [ppm] = 2.49 (s, 3H), 2.37 (s, 3H).

**H. 4-Ethylpyrimidin-2-ol 1-oxid Hydrochlorid**

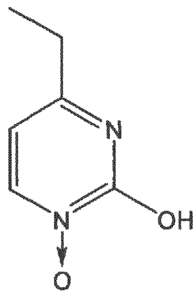

**1,1-Dimethoxypentan-3-on**

(T. Harayama, H. Cho und Y. Inubushi, Chem. Pharm. Bull. 1978, 26, 1201-1214)

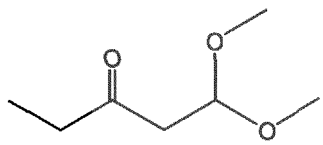

In einem Mehrhalskolben mit Innenthermometer und KPG-Rührer wurden 0.96 mol (89 g) Propionsäurechlorid vorgelegt und mit Salz/Eis Kältemischung gekühlt. Es wurden portionsweise 0.82 mol (110 g) Aluminiumchlorid zugegeben, die Mischung wurde für 10 min kräftig gerührt und mit 50 ml Chloroform versetzt. Dann wurden innerhalb von ca. 1 h 0.93 mol (100 g) Vinylbromid in kleinen Portionen zugegeben (maximale Innentemperatur 14°C). Es wurde noch 1 h auf Eis gerührt, dann wurde die Reaktionsmischung auf 500 g Eis gegossen und mehrfach mit insgesamt 1 l Chloroform extrahiert. Die vereinigten organischen Phasen wurden 4x mit je 1 l Wasser gewaschen, mit Natriumsulfat getrocknet und das Chloroform wurde am Rotationsverdampfer abdestilliert. Der Rückstand wurde bei 80°C Wasserbadtemperatur und 16 mbar am Rotationsverdampfer destilliert (Kopftemperatur ca. 47°C), wobei 118 g Zwischenprodukt (instabil, Lagerung -18°C) erhalten wurden.

118 g Zwischenprodukt wurden in 600 ml wasserfreiem Methanol gelöst vorgelegt und auf Eis gekühlt. Es wurden 1.03 mol (55.65 g) Natriummethoxid gelöst in 360 ml wasserfreiem Methanol in 30 min zugetropft und das Gemisch wurde bei RT weitere 18 h gerührt. Dann wurde vom entstandenen Salz abfiltriert, mit wenig trockenem Methanol nachgewaschen und das Filtrat am Rotationsverdampfer eingeengt. Der Rückstand wurde bei 75°C Wasserbadtemperatur und 4 mbar am Rotationsverdampfer destilliert (Kopftemperatur 40-52°C). Es wurden 61.4 g eines Produktgemisches erhalten, das 62% (m/m) der Titelverbindung (entsprechend 38 g) enthielt.
1 H-NMR (DMSO-$d_6$, 400 MHz): $\delta$ [ppm] = 4.74 (t, 1 H), 3.23 (s, 6H), 2.70 (d, 2H), 2.45 (q, 2H), 0.90 (t, 3H) (Titelverbindung, 62% m/m in Produktgemisch); $\delta$ = 7.69 (d, 1 H), 5.61 (d, 1 H), 3.69 (s, 3H), 2.48 (q, 2H), 0.96 (t, 3H) ((1*E*)-1-Methoxypent-1-en-3-on, 38% in Produktgemisch).

**4-Ethylpyrimidin-2-ol 1-oxid Hydrochlorid**

0.388 mol (25.9 g) Hydroxyharnstoff wurden in 195 ml 2 M HCl gelöst, 80 ml Methanol zugegeben und unter Kühlung 0.388 mol (56.7 g) 1,1-Dimethoxypentan-3-on (62% Anteil in Produktgemisch) zugetropft, wobei die Innentemperatur auf -6 bis -7°C gehalten wurde. Die Lösung wurde im Eisbad auf Raumtemperatur aufgetaut und 1 h gerührt, dann zur Trockne eingeengt. Der Rückstand wurde mit 100 ml Aceton aufgeschlämmt, das Gemisch im Eis/Ethanolbad auf unter 0°C gekühlt, der Feststoff abfiltriert und mit wenig eiskaltem Aceton nachgewaschen. Nach dem Trocknen wurden 32.6 g Rohprodukt erhalten. Das Rohprodukt wurde mit 200 ml Ethanol zum Sieden erhitzt, heiss filtriert und langsam auf -18°C gekühlt. Der ausgefallene Feststoff wurde abfiltriert, mit wenig Ethanol gewaschen und getrocknet. Man erhielt 11.7 g der Titelverbindung.
IR (in Substanz, $cm^{-1}$): 3115, 3038, 2936, 2678, 2518, 1753, 1606, 1585, 1516, 1465, 1403, 1381, 1301, 1229, 1184, 1134, 1109, 1053, 1002, 896, 803, 769, 736,680,605,540,513,494,474.
CHN-Elementaranalyse: C, 40.72; H, 5.03; N, 15.32.
LC-MS: 141 (M+H).
1H-NMR (DMSO-$d_6$, 400 MHz): $\delta$ [ppm] = 8.92 (d, 1H), 6.70 (d, 1H), 2.73 (q, 2H); 1.19 (t, 3H). Aus dem NMR-Spektrum wurde abgeschätzt, dass das Produkt <3% des Regioisomeren 6-Ethylpyrimidin-2-ol 1-oxid Hydrochlorid enthielt.

**I. 6-Ethyl-4-methylpyrimidin-2-ol 1-oxid Hydrochlorid**

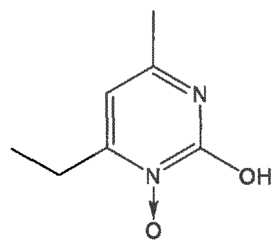

0.12 mol (9.13 g) Hydroxyharnstoff wurden in 50 ml 2 M HCl gelöst, 20 ml Methanol zugegeben und unter Kühlung bei ca. -15°C 0.10 mol (11.41 g) 2,4-Hexandion zugetropft. Es wurden weitere 30 ml Wasser und 10 ml Methanol zugegeben. Die zweiphasige Reaktionsmischung wurde langsam auf Raumtemperatur erwärmen lassen und noch 2 h bei Raumtemperatur gerührt, dann zur Trockne eingeengt. Der Rückstand wurde mit 50 ml Aceton aufgeschlämmt, das Gemisch im Eis/Ethanolbad gekühlt, der Feststoff abfiltriert und mit wenig eiskaltem Aceton nachgewaschen. Nach dem Trocknen wurden 7.88 g Rohprodukt erhalten, die aus 45 ml Ethanol umkristallisiert wurden, wobei zunächst ein unlöslicher Salzanteil heiss abfiltriert wurde und das Produkt anschliessend aus dem Filtrat bei -18°C auskristallisierte. Es wurden 3.0 g Produkt erhalten, die erneut aus 28 ml Ethanol umkristallisiert wurden. Es wurden schliesslich 2.26 g der Titelverbindung erhalten.
IR (in Substanz, $cm^{-1}$): 3093, 2997, 2945, 2679, 2555, 1805, 1741, 1601, 1571, 1508, 1435, 1401, 1370, 1327, 1290, 1253, 1213, 1157, 1103, 1049, 903, 849, 811,766,742,701,669,626,607,582,512,494.
LC-MS (m/z): 155.7 (M+H).
1 H-NMR (DMSO-$d_6$, 400 MHz): $\delta$ [ppm] = 6.78 (s, 1 H), 2.88 (q, 2H), 2.46 (s, 3H), 1.21 (t, 3H). Aus dem NMR-Spektrum wurde abgeschätzt, dass das Produkt ca. 6.6% des Regioisomeren 4-Ethyl-6-methylpyrimidin-2-ol 1-oxid Hydrochlorid enthielt.

**J. 4-Methyl-6,7-dihydro-5H-cyclopenta[*d*]pyrimidin-2-ol 1-oxid Hydrochlorid**

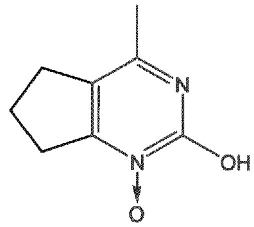

0.20 mol (15.21 g) Hydroxyharnstoff wurden in 200 ml 1 M HCl gelöst, 200 ml Methanol zugegeben und 0.20 mol (25.23 g) 2-Acetylcyclopentanon zugetropft, noch 1 h gerührt und dann die Lösung am Rotationsverdampfer zur Trockne eingeengt. Der Rückstand wurde mit 100 ml Aceton aufgeschlämmt, der Feststoff abfiltriert und mit Aceton nachgewaschen. Nach dem Trocknen wurden 12.41 g Rohprodukt 1 erhalten, das mit 1200 ml Isopropanol in der Siedehitze gelöst und heiss filtriert wurde. Das Filtrat wurde zur Trockne eingeengt und es wurden 8.49 g Rohprodukt 2 erhalten. Dieses wurde in der Siedehitze in 200 ml Ethanol gelöst und mit 200 ml Tetrahydrofuran versetzt. Der ausgefallene Feststoff wurde abfiltriert und getrocknet. Es wurden 5.63 g eines Produkts erhalten, das 91.5% der Titelverbindung und 8.5% Ammoniumchlorid enthielt.
IR (in Substanz, $cm^{-1}$): 3133, 3042, 2841, 2751, 2480, 1730, 1613, 1590, 1493, 1404, 1374, 1314, 1289, 1221, 1134, 1062, 1044, 1020, 972, 938, 894, 868, 822, 740, 707, 637, 575, 552, 525, 509.
LC-MS (m/z): 167.5 (M+H).
CHN-Elementaranalyse: C, 43.93; H, 6.07; N, 13.41.
Chlorid-Gehalt: 21.7% (m/m)
1H-NMR (DMSO-$d_6$, 400 MHz): $\delta$ [ppm] = 3.22 (t, 2H), 2.82 (t, 2H), 2.42 (s, 3H), 2.13 (quintett, 2H). Aus dem NMR-Spektrum wurde abgeschätzt, dass das Produkt ca. 6% des Regioisomeren 4-Methyl-6,7-dihydro-5*H*-cyclopenta[dlpyrimidin-2-ol 3-oxid Hydrochlorid enthielt.

**K. 4-Methyl-5,6,7,8-tetrahydrochinazolin-2-ol 3-oxid Hydrochlorid**

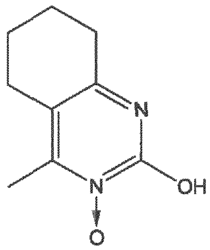

0.08 mol (6.08 g) Hydroxyharnstoff wurden in 40 ml 2 M HCl gelöst, 40 ml Methanol zugegeben und unter Kühlung bei ca. -15°C 0.08 mol (11.21 g) 2-Acetylcyclohexanon zugetropft, noch 1 h gerührt und dabei auf 20°C erwärmt. Dieser Ansatz wurde insgesamt sechsmal durchgeführt. Die vereinten Reaktionsmischungen wurden dann am Rotationsverdampfer eingeengt. Der Rückstand wurde mit Aceton aufgeschlämmt, der Feststoff abfiltriert und mit Aceton nachgewaschen. Nach dem Trocknen wurden 36.97 g Rohprodukt 1 erhalten, das mit 250 ml Ethanol aufgeschlämmt und heiss filtriert wurde. Das Filtrat wurde zur Trockne eingeengt und es wurden 20.87 g Rohprodukt 2 erhalten. Dieses wurde in der Siedehitze in 500 ml Ethanol gelöst, heiss filtriert, und das Filtrat wurde mit 800 ml Tetrahydrofuran versetzt. Der ausgefallene Feststoff wurde abfiltriert und getrocknet. Es wurden 14.3 g eines Produkts erhalten, das 87% der Titelverbindung und 13% Ammoniumchlorid enthielt.
IR (in Substanz, $cm^{-1}$): 3135, 3044, 2937, 2875, 2805, 2706, 2426, 1743, 1572, 1501, 1443, 1403, 1345, 1288, 1260, 1235, 1150, 1122, 1086, 1041, 908, 883, 824,740,707,669,643,605,546,514.
CHN-Elementaranalyse: C, 43.63; H, 6.08; N, 14.66.
Chlorid-Gehalt: 22.2% (m/m)
1H-NMR (DMSO-$d_6$, 400 MHz): $\delta$ [ppm] = 2.76 (m, 2H), 2.53 (s, 3H), 2.49 (m, 2H), 1.70 (m, 4H). Aus dem NMR-Spektrum wurde abgeschätzt, dass das Produkt ca. 5% des Regioisomeren 4-methyl-5,6,7,8-Tetrahydrochinazolin-2-ol 1-oxid

Hydrochlorid enthielt.

**L. 4-(Propan-2-yl)pyrimidin-2-ol 1-oxid Hydrochlorid**

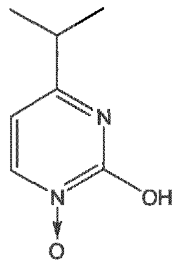

0.187 mol (14.22 g) Hydroxyharnstoff wurden in 200 ml 1 M HCl gelöst und unter Kühlung 0.187 mol (30 g) 1,1-Dimethoxy-4-methylpentan-3-on (E.E. Royals und K.C. Brannock, J. Am. Chem. Soc. 1953, 75, 2050-2053) zugetropft, wobei die Innentemperatur auf 0-1 °C gehalten wurde. Die zweiphasige Mischung wurde im Eisbad auf Raumtemperatur aufgetaut und 12 h gerührt, dann zur Trockne eingeengt. Der Rückstand wurde mit 100 ml Aceton aufgeschlämmt, das Gemisch im Eis/Ethanolbad gekühlt, der Feststoff abfiltriert und mit wenig eiskaltem Aceton nachgewaschen. Nach dem Trocknen wurden 15.79 g Rohprodukt 1 erhalten. 11.04 g des Rohprodukts 1 wurden mit 141 ml Ethanol zum Sieden erhitzt und ausgefallener Feststoff nach dem Abkühlen abfiltriert. Das Filtrat wurde erneut zur Trockne eingeengt und 8.49 g Rohprodukt 2 erhalten, welches in 80 ml Ethanol zum Sieden erhitzt und heiss filtriert wurde. Das Filtrat wurde langsam auf Raumtemperatur und über Nacht auf -18°C abgekühlt. Der ausgefallene Feststoff wurde abfiltriert und nach dem Trocknen wurden 2.18 g der Titelverbindung erhalten.
IR (in Substanz, $cm^{-1}$): 2971, 2585, 1815, 1748, 1598, 1572, 1513, 1463, 1390, 1305, 1230, 1186, 1163, 1132, 1049, 986, 934, 901, 815, 773, 749, 719, 681, 616, 582, 518, 498, 484, 478.
CHN-Elementaranalyse: C, 43.54; H, 6.10; N, 14.50
LC-MS (m/z): 155.5 (M+H).
1 H-NMR (DMSO-$d_6$, 400 MHz): $\delta$ [ppm] = 8.88 (d, 1 H), 6.68 (d, 1 H), 3.02 (heptett, 1 H); 1.20 (d, 6H)

**M. 4-Ethyl-6-methylpyrimidin-2-ol 1-oxid Hydrochlorid**

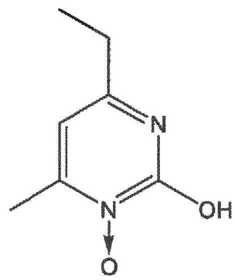

Aus der Synthese des Regioisomeren 6-Ethyl-4-methylpyrimidin-2-ol 1-oxid Hydrochlorid (Beispiel I.) wurden verschiedene Mutterlaugen aus der Umkristallisation vereinigt und zur Trockne eingeengt. Insgesamt 22.14 g dieses die Titelverbindung zu ca. 50% enthaltenden Produktgemischs wurden in 140 ml Ethanol zum Sieden erhitzt, heiss filtriert und zur Trockne eingeengt. 20.96 g Rückstand wurden aus 90 ml Ethanol umkristallisiert, wobei zunächst 0.82 g unlöslicher Anteil heiss abgetrennt und dann langsam von der Siedehitze auf -18°C gekühlt wurde. 17.25 g Niederschlag wurden anschliessend aus 150 ml Ethanol / 70 ml Tetrahydrofuran entsprechend umkristallisiert, wobei 6.05 g überwiegend 6-Ethyl-4-methylpyrimidin-2-ol 1-oxid Hydrochlorid enthaltender Feststoff abgetrennt wurde. Das Filtrat wurde zur Trockne eingeengt und 9.53 g Rückstand wurden aus 180 ml Isopropanol umkristallisiert, die erhaltenen 7.57 g Produktgemisch (63% Anreicherung) wurden entsprechend noch mehrfach aus Isopropanol umkristallisiert. Es wurden schliesslich 0.69 g der Titelverbindung erhalten.
IR (in Substanz, $cm^{-1}$): 3077, 2853, 2685, 2550, 1745, 1608, 1568, 1514, 1461, 1416, 1370, 1323, 1304, 1249, 1211, 1160, 1142, 1104, 1069, 1028, 994, 936, 887, 849, 767, 746, 707, 685, 625, 599, 567, 525, 500.

LC-MS (m/z): 155 (M+H).
1H-NMR ($DMSO-d_6$, 400 MHz): δ [ppm] = 6.83 (s, 1H), 2.70 (q, 2H), 2.55 (s, 3H), 1.20 (t, 3H). Aus dem NMR-Spektrum wurde abgeschätzt, dass das Produkt ca. 13% des Regioisomeren 6-Ethyl-4-methylpyrimidin-2-ol 1-oxid Hydrochlorid enthielt.

**N. 4-tert-Butylpyrimidin-2-ol 1-oxid Hydrochlorid**

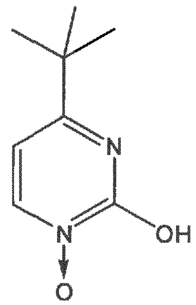

Die Synthese der Vorstufe 1,1-Dimethoxy-4,4-dimethylpentan-3-on wurde analog E.E. Royals und K.C. Brannock (J. Am. Chem. Soc. 1953, 75, 2050-2053) durchgeführt, wobei eine Mischung aus ca. 20% der gewünschten Vorstufe und 80% des Nebenproduktes 1-Methoxy-4,4-dimethylpent-1-en-3-on erhalten wurde. 32.8 g dieser Mischung wurden mit 33.9 mmol (5.63 g) N-Benzyloxyharnstoff, 51 ml Methanol, 1.7 ml Wasser und 4.06 ml Schwefelsäure versetzt (analog M. Yamaguchi et al., J. Inorg. Biochemistry 2006, 100, 260-269). Es wurde bei Raumtemperatur unter portionsweiser Zugabe von gesamt 153 mmol (25 g) N-Benzyloxyharnstoff 5 h gerührt, bis N-Benzyloxyharnstoff im Reaktionsgemisch nachgewiesen werden konnte (DC Hexan/Ethylacetat 2/1, 1 % Essigsäure). Die Reaktionsmischung wurde zur Trockne eingeengt, der Rückstand in Wasser/Dichlormethan aufgenommen, die Wasserphase mit gesättigter Natriumcarbonatlösung auf pH 9.3 eingestellt und dreimal mit Dichlormethan extrahiert. Die vereinigten organischen Phasen wurden mit Wasser gewaschen, über Natriumsulfat getrocknet und zur Trockne eingeengt. Es wurden 51.8 g eines Rohprodukts erhalten, das mit Cyclohexan/Ethylacetat über Kieselgel chromatografiert wurde. Es wurden 46.4 mmol (12.0 g) gereinigtes Zwischenprodukt erhalten, das in 240 ml Methanol gelöst und mit 0.93 g 10% Pd/C 3.5 h mit Wasserstoff hydriert wurde. Es wurde über Celite abfiltriert, das Filtrat mit 50 ml 1 M HCl versetzt und zur Trockne eingeengt. Das Rohprodukt wurde in 100 ml Wasser suspendiert, von unlöslichen Bestandteilen abfiltriert und das Filtrat eingeengt. Nach dem Trocknen wurden 8.15 g (39.8 mmol, 5.0% Ausbeute über 3 Stufen) der Titelverbindung erhalten.
IR (in Substanz, $cm^{-1}$): 2850, 2482, 1758, 1605, 1565, 1517, 1494, 1467, 1389, 1377, 1310, 1264, 1190, 1118, 1086, 887, 828, 751, 730, 703.
1 H-NMR ($DMSO-d_6$, 400 MHz): δ [ppm] = 8.66 (d, 1 H), 6.61 (d, 1 H), 1.27 (s, 9H)

**O. 5,6-Dimethylpyrimidin-2-ol 1-oxid Hydrochlorid**

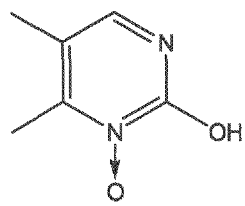

Die Synthese der Vorstufe 4,4-Dimethoxy-3-methylbutan-2-on wurde analog E.E. Royals und K.C. Brannock (J. Am. Chem. Soc. 1953, 75, 2050-2053) durchgeführt und ergab eine Mischung aus 58% der gewünschten Vorstufe, 24% des Nebenproduktes 1,1-Dimethoxy-pentan-3-on sowie ca. 18% Eliminierungsprodukt. 102 g dieser Mischung (0.405 mol 4,4-Dimethoxy-3-methylbutan-2-on) wurden in 30 ml Methanol gelöst und zu 0.698 mol N-Hydroxyharnstoff in 400 ml 2M HCl zugetropft, wobei die Innentemperatur auf -10 bis -4°C gehalten wurde. Die Lösung wurde auf Raumtemperatur aufgetaut und 1 h gerührt, dann zur Trockne eingeengt. Der Rückstand wurde mit 200 ml Aceton aufgeschlämmt, der Feststoff abfiltriert und mit wenig eiskaltem Aceton nachgewaschen. Nach dem Trocknen wurde das Rohprodukt

mit 150 ml Ethanol zum Sieden erhitzt, heiss filtriert, auf 50 ml eingeengt und auf -18°C gekühlt. Der ausgefallene Feststoff wurde abfiltriert, mit wenig Ethanol gewaschen und getrocknet. 20 g dieses Feststoffs wurden erneut aus 100 ml Ethanol umkristallisiert, es wurden schliesslich 10 g (12 % Ausbeute) der Titelverbindung erhalten (Gehalt 85% der Titelverbindung sowie 13% Ammoniumchlorid).
IR (in Substanz, $cm^{-1}$): 2569, 2539, 1726, 1628, 1589, 1503, 1453, 1378, 1336, 1255, 1220, 1157, 1141, 1120, 1021, 919, 828, 755, 734, 713.
1 H-NMR (DMSO-$d_6$, 400 MHz): $\delta$ [ppm] = 8.98 (s, 1 H); 2.50 (s, 3H); 2.08 (s, 3H)

P. 6-n-Propylpyrimidin-2-ol 1-oxid Hydrochlorid

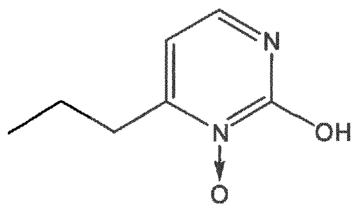

Die Synthese der Vorstufe 1,1-Dimethoxyhexan-3-on wurde analog E.E. Royals und K.C. Brannock (J. Am. Chem. Soc. 1953, 75, 2050-2053) durchgeführt und ergab eine Mischung aus 75% der gewünschten Vorstufe, 13% des Nebenproduktes 3-(Dimethoxymethyl)pentan-2-on sowie ca. 12% Eliminierungsprodukte. 29.2 g dieser Mischung (0.137 mol 1,1-Dimethoxyhexan-3-on) wurden mit 0.164 mol (27.3 g) N-Benzyloxyharnstoff, 150 ml Methanol und 20 ml Schwefelsäure versetzt (analog M. Yamaguchi et al., J. Inorg. Biochemistry 2006, 100, 260-269). Es wurde bei Raumtemperatur gerührt, dann wurden weitere 0.0164 mmol (2.73 g) N-Benzyloxyharnstoff zugegeben und noch 1 h auf 50°C geheizt. Das Gemisch wurde zur Trockne eingeengt, der Rückstand in Wasser/Dichlormethan aufgenommen, die Wasserphase mit gesättigter Natriumcarbonatlösung auf pH 11 eingestellt und dreimal mit Dichlormethan extrahiert. Die vereinigten organischen Phasen wurden mit Wasser gewaschen, über Magnesiumsulfat getrocknet und zur Trockne eingeengt. Es wurden 40.3 g Rohprodukt erhalten, das mit Cyclohexan/Ethylacetat über Kieselgel chromatografiert wurde. 0.026 mol (6.3 g) des gereinigten Zwischenproduktes wurden in 100 ml Methanol gelöst und mit 0.53 g 10% Pd/C 0.5 h mit Wasserstoff hydriert. Es wurde über Celite abfiltriert, das Filtrat auf 50 ml eingeengt, mit 50 ml 1 M HCl versetzt und zur Trockne eingeengt. Das Rohprodukt wurde aus 50 ml 2-Propanol und 200 ml Diethylether umkristallisiert. Es wurden 3.46 g (18.2 mmol, 13% Ausbeute über 2 Stufen) der Titelverbindung erhalten.
IR (in Substanz, $cm^{-1}$): 2591, 2536, 2477, 1770, 1736, 1608, 1580, 1311, 1194, 1185, 1130, 1115, 1082, 1001, 904, 892, 823, 787, 736, 680.
1H-NMR (DMSO-$d_6$, 400 MHz): ö [ppm] = 8.98 (d, 1H), 6.75 (d, 1H), 2.74 (t, 2H); 1.69 (hextett, 2H); 0.93 (t, 3H)

__**Eisen-Komplexverbindungen (Beispiele)**__

**Beispiel 1**

**Tris-(Pyrimidin-2-ol 1-oxid)-eisen(III)-Komplex**

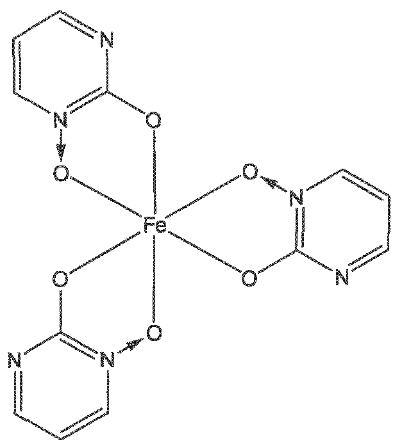

114 mmol (16.93 g) Pyrimidin-2-ol 1-oxid Hydrochlorid wurden in 150 ml Wasser gelöst und 38 mmol (10.27 g) in 15 ml Wasser gelöstes $FeCl_3*6H_2O$ zugegeben. Die Lösung wurde mit ca. 90 ml 2 M NaOH auf pH 6.3 eingestellt und 0.5 h nachgerührt. Das Produkt wurde abfiltriert, mit Wasser gewaschen und bei 50°C im Vakuumtrockenschrank getrocknet. Man erhielt 14.2 g der Titelverbindung.
IR (in Substanz, $cm^{-1}$): 3082, 3054, 1596, 1506, 1431, 1366, 1278, 1197, 1136, 1108, 1055, 907, 798, 765, 613, 554, 513.
CHN-Elementaranalyse: C, 35.77; H, 2.78; N, 20.23.
ESI-MS: 278.3 ($FeL_2^+$); 390.4 ($M+H^+$); 412.4 ($M+Na^+$).
Fe-Gehalt: 13.61% [m/m]

**Beispiel 2**

**Tris-(4-Methylpyrimidin-2-ol 1-oxid)-eisen(III)-Komplex**

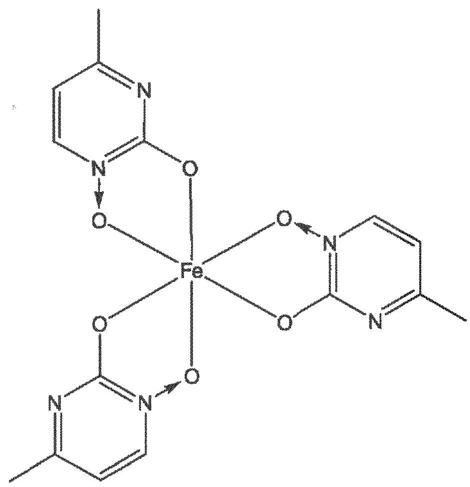

21 mmol (3.76 g, ca. 90% Reinheit) 4-Methylpyrimidin-2-ol 1-oxid Hydrochlorid wurden in 15 ml Wasser gelöst und 7.0 mmol (1.89 g) in 5 ml Wasser gelöstes $FeCl_3*6H_2O$ zugegeben. Die Lösung wurde mit ca. 44 ml 1 M NaOH auf pH 5.85 eingestellt und 0.5 h nachgerührt. Das Produkt wurde abfiltriert, mit Wasser gewaschen und bei 50°C im Vakuumtrockenschrank getrocknet. Man erhielt 3.03 g der Titelverbindung.
IR (in Substanz, $cm^{-1}$): 3400, 3074, 1602, 1545, 1503, 1425, 1378, 1339, 1249, 1201, 1145, 1110, 1032, 954, 805, 762, 645, 600.
CHN-Elementaranalyse: C, 38.42; H, 4.10; N, 18.11.
ESI-MS: 306.4 ($FeL_2^+$); 432.4 ($M+H^+$).
Fe-Gehalt: 12.15% [m/m]

**Beispiel 3**

**Tris-(4,6-Dimethylpyrimidin-2-ol 1-oxid)-eisen(III)-Komplex**

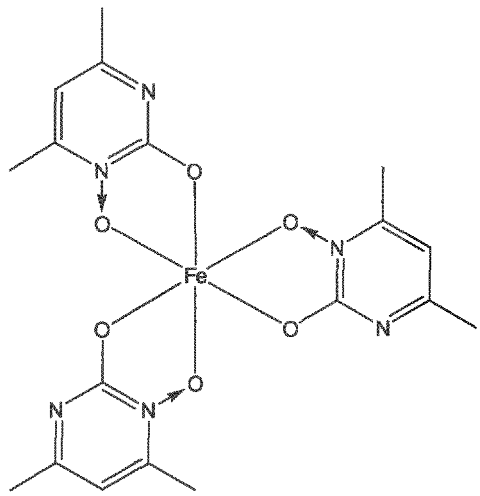

120 mmol (21.19 g) 4,6-Dimethylpyrimidin-2-ol 1-oxid Hydrochlorid wurden in 15 ml Wasser gelöst und 40 mmol (10.81 g) in 10 ml Wasser gelöstes $FeCl_3$*$6H_2O$ zugegeben. Die Lösung wurde mit ca. 238 ml 1 M NaOH auf pH 5.90 eingestellt und 0.5 h nachgerührt. Das Produkt wurde abfiltriert, mit Wasser gewaschen und bei 50°C im Vakuumtrockenschrank getrocknet. Man erhielt 18.66 g der Titelverbindung.
IR (in Substanz, $cm^{-1}$): 3596, 3441, 3077, 1604, 1551, 1511, 1441, 1393, 1380, 1360, 1320, 1153, 1097, 1029, 875, 856, 798, 651, 564, 524, 486. CHN-Elementaranalyse: C, 44.27; H, 4.22; N, 17.35.
ESI-MS: 334.4 ($FeL_2^+$); 474.5 ($M+H^+$); 496.6 ($M+Na^+$).
Fe-Gehalt: 11.33% [m/m]
Kein Schmelzpunkt, ab ca. 230°C setzt eine exotherme Zersetzung ein.

**Beispiel 4**

**Tris-(4,6-Diethylpyrimidin-2-ol 1-oxid)-eisen(III)-Komplex**

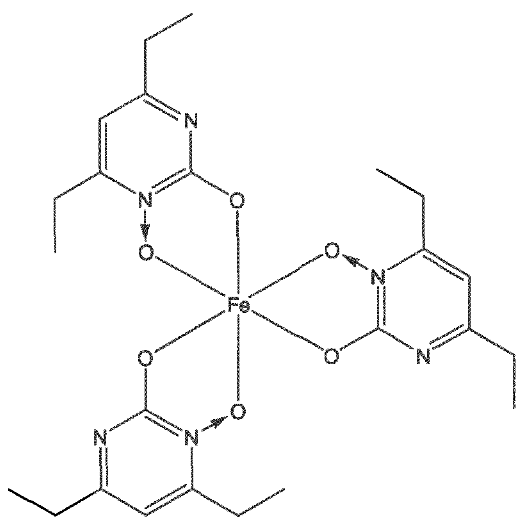

14 mmol (6.1 g, ca. 48% Reinheit, 52% Ammoniumchlorid) 4,6-Diethylpyrimidin-2-ol 1-oxid Hydrochlorid wurden

in 15 ml Wasser gelöst und 4.67 mmol (1.26 g) in 2 ml Wasser gelöstes $FeCl_3*6H_2O$ zugegeben. Die Lösung wurde mit 27.7 ml 1 M NaOH auf pH 5.85 eingestellt und 0.5 h nachgerührt. Das Produkt wurde abfiltriert, mit Wasser gewaschen und bei 50°C im Vakuumtrockenschrank getrocknet. Man erhielt 2.57 g der Titelverbindung.
IR (in Substanz, $cm^{-1}$): 3593, 3378, 3087, 2969, 2934, 2880, 1602, 1549, 1510, 1460, 1406, 1328, 1255, 1235, 1146, 1108, 1073, 1027, 964, 903, 863, 807, 764, 701, 672, 645, 619, 578, 522.
CHN-Elementaranalyse: C, 49.23; H, 6.03; N, 14.43.
Fe-Gehalt: 10.05 % [m/m]

**Beispiel 5**

**Tris-(4-Methyl-6-(2-methylpropyl)pyrimidin-2-ol 1-oxid)-eisen(III)-Komplex**

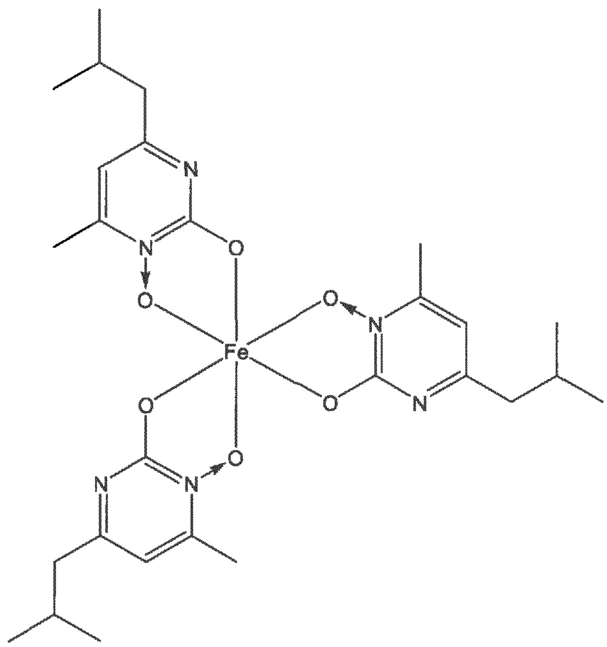

15 mmol (5.74 g, ca. 58% Reinheit, 42% Ammoniumchlorid) 4-Methyl-6-(2-methylpropyl)pyrimidin-2-ol 1-oxid Hydrochlorid wurden in 25 ml Wasser gelöst und 5.0 mmol (1.35 g) in 2 ml Wasser gelöstes $FeCl_3*6H_2O$ zugegeben. Die Lösung wurde mit 29.2 ml 1 M NaOH auf pH 5.88 eingestellt und 0.5 h nachgerührt. Das Produkt wurde abfiltriert, mit Wasser gewaschen und bei 50°C im Vakuumtrockenschrank getrocknet. Man erhielt 3.00 g der Titelverbindung.
IR (in Substanz, $cm^{-1}$): 2959, 2929, 2872, 1598, 1549, 1513, 1462, 1434, 1400, 1355, 1289, 1233, 1151, 1122, 1102, 1034, 990, 928, 879, 853, 799, 769, 701, 648, 606, 575.
CHN-Elementaranalyse: C, 53.71; H, 6.40; N, 13.92.
Fe-Gehalt: 9.29% [m/m]

**Beispiel 6**

**Tris-(4,5,6-Trimethylpyrimidin-2-ol 1-oxid)-eisen(III)-Komplex**

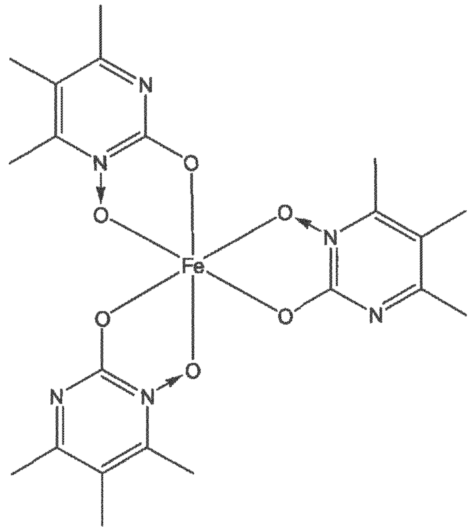

10.2 mmol (2.38 g, ca. 82% Reinheit, 18% Ammoniumchlorid) 4,5,6-Trimethylpyrimidin-2-ol 1-oxid Hydrochlorid wurden in 5 ml Wasser gelöst und 3.4 mmol (0.93 g) in 1 ml Wasser gelöstes $FeCl_3*6H_2O$ zugegeben. Die Lösung wurde mit 19 ml 1 M NaOH auf pH 6.04 eingestellt und 0.5 h nachgerührt. Das Produkt wurde abfiltriert, mit Wasser gewaschen und bei 50°C im Vakuumtrockenschrank getrocknet. Man erhielt 1.72 g der Titelverbindung.
IR (in Substanz, $cm^{-1}$): 3424, 2925, 1593, 1510, 1439, 1377, 1234, 1188, 1160, 1109,999,935,869,803,763,720,657,613,561.
CHN-Elementaranalyse: C, 45.43; H, 5.80; N, 14.12.
Fe-Gehalt: 10.68% [m/m]

**Beispiel 7**

**Tris-(5-Chloro-4,6-dimethylpyrimidin-2-ol 1-oxid)-eisen(III)-Komplex**

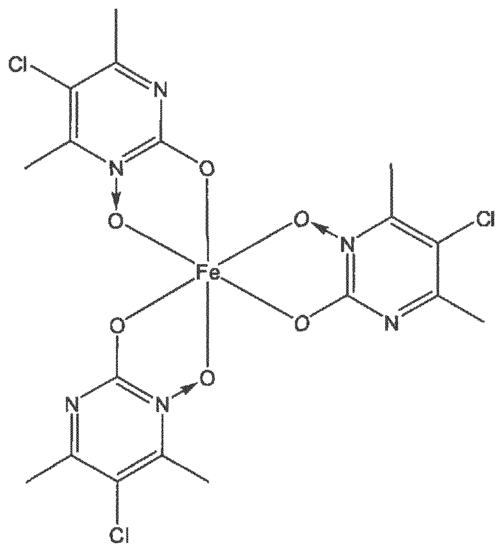

6.73 mmol (3.8 g, ca. 37% Reinheit, 63% Ammoniumchlorid) sowie 0.77 mmol (0.31 g, ca. 53% Reinheit, 47% Ammoniumchlorid) 5-Chloro-4,6-dimethylpyrimidin-2-ol 1-oxid Hydrochlorid wurden in 25 ml Wasser gelöst und 2.5 mmol (0.68 g) in 2 ml Wasser gelöstes $FeCl_3*6H_2O$ zugegeben. Die Lösung wurde mit 14.8 ml 1 M NaOH auf pH 5.96 eingestellt und 0.5 h nachgerührt. Das Produkt wurde abfiltriert, mit Wasser gewaschen und bei 50°C im Vakuumtrockenschrank getrocknet. Man erhielt 1.37 g der Titelverbindung.
IR (in Substanz, $cm^{-1}$): 2929, 2363, 1688, 1589, 1509, 1431, 1390, 1375, 1196, 1169, 1092, 1016, 968, 847, 759, 694,

667, 553.
CHN-Elementaranalyse: C, 37.01; H, 2.83; N, 14.47.
Fe-Gehalt: 9.88% [m/m]

**Beispiel 8**

**Tris-(4-Ethylpyrimidin-2-ol 1-oxid)-eisen(III)-Komplex**

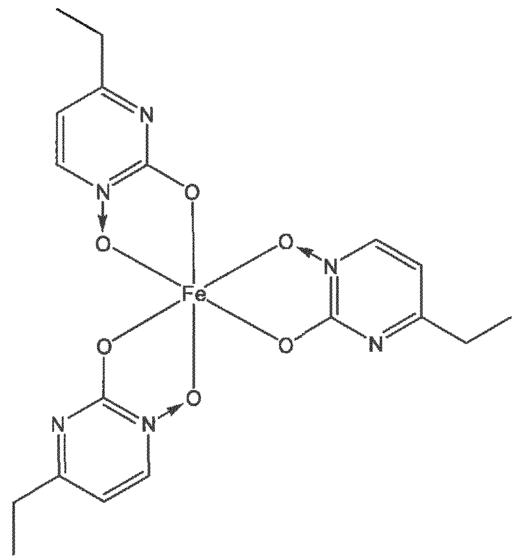

63 mmol (11.13 g) 4-Ethylpyrimidin-2-ol 1-oxid Hydrochlorid wurden in 20 ml Wasser gelöst und 21 mmol (5.68 g) in 5 ml Wasser gelöstes $FeCl_3$*$6H_2O$ zugegeben. Die Lösung wurde mit 125.6 ml 1 M NaOH auf pH 6.17 eingestellt und 0.5 h nachgerührt. Das Produkt wurde abfiltriert, mit Wasser gewaschen und bei 50°C im Vakuumtrockenschrank getrocknet. Man erhielt 9.52 g der Titelverbindung.
IR (in Substanz, $cm^{-1}$): 3083, 2974, 2936, 2876, 1596, 1543, 1511, 1460, 1428, 1313, 1249, 1194, 1143, 1107, 1079, 1056, 991, 948, 811, 770, 748, 696, 639, 598, 531, 502.
CHN-Elementaranalyse: C, 44.67; H, 4.52; N, 17.36.
Fe-Gehalt: 11.40% [m/m]

**Beispiel 9**

**Tris-(6-Ethyl-4-methylpyrimidin-2-ol 1-oxid)-eisen(III)-Komplex**

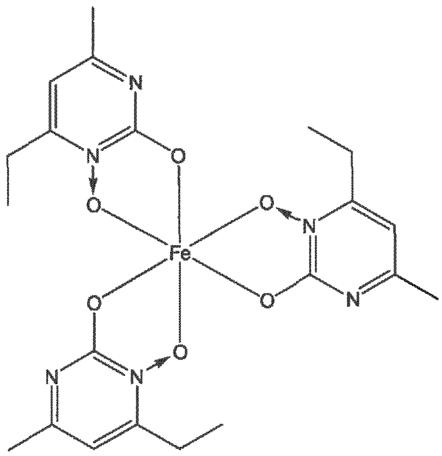

11.4 mmol (2.17 g) 6-Ethyl-4-methylpyrimidin-2-ol 1-oxid Hydrochlorid wurden in 5 ml Wasser gelöst und 3.8 mmol (1.03 g) in 2 ml Wasser gelöstes $FeCl_3*6H_2O$ zugegeben. Die Lösung wurde mit 22.7 ml 1 M NaOH auf pH 6.3 eingestellt und 0.5 h nachgerührt. Das Produkt wurde abfiltriert, mit Wasser gewaschen und bei 50°C im Vakuumtrockenschrank getrocknet. Man erhielt 1.89 g der Titelverbindung.
IR (in Substanz, $cm^{-1}$): 3515, 3080, 2974, 2938, 1598, 1550, 1516, 1461, 1427, 1401, 1317, 1257, 1229, 1149, 1104, 1060, 1035, 985, 918, 851, 813, 768, 681, 651,557,522.
CHN-Elementaranalyse: C, 47.48; H, 5.44; N, 15.81.
Fe-Gehalt: 10.32% [m/m]
Chlorid-Gehalt: 0.0% [m/m]

**Beispiel 10**

**Tris-(4-Methyl-6,7-dihydro-5*H*-cyclopenta[d]pyrimidin-2-ol 1-oxid)-eisen(III)-Komplex**

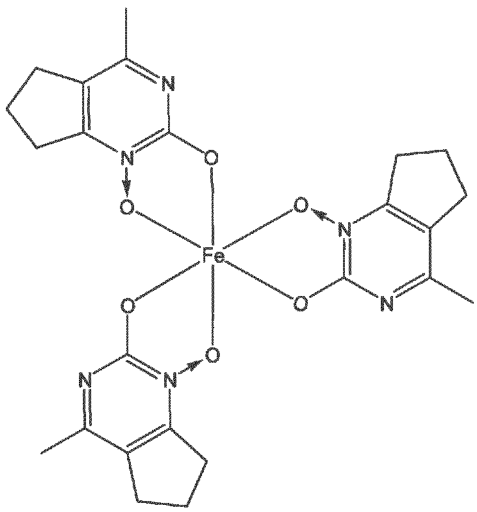

21 mmol (4.73 g) 4-Methyl-6,7-dihydro-5*H*-cyclopenta[*d*]pyrimidin-2-ol 1-oxid Hydrochlorid (Gehalt ca. 90%) wurden in 30 ml Wasser gelöst und 7.0 mmol (1.89 g) in 5 ml Wasser gelöstes $FeCl_3*6H_2O$ zugegeben. Die Lösung wurde mit 41.6 ml 1 M NaOH auf pH 5.96 eingestellt und 0.5 h nachgerührt. Das Produkt wurde abfiltriert, mit Wasser gewaschen und bei 50°C im Vakuumtrockenschrank getrocknet. Man erhielt 3.8 g der Titelverbindung.
IR (in Substanz, $cm^{-1}$): 2918, 2361, 2326, 1599, 1571, 1499, 1429, 1382, 1359, 1311, 1279, 1232, 1208, 1173, 1100, 1067, 1043, 1013, 970, 945, 904, 881, 836, 761, 733, 663, 566, 528, 499.
CHN-Elementaranalyse: C, 50.36; H, 4.98; N, 14.88.
Fe-Gehalt: 9.71% [m/m]

Chlorid-Gehalt: 1.05% [m/m]

**Beispiel 11**

**Tris-(4-Methyl-5,6,7,8-tetrahydrochinazolin-2-ol 3-oxid)-eisen(III)-Komplex**

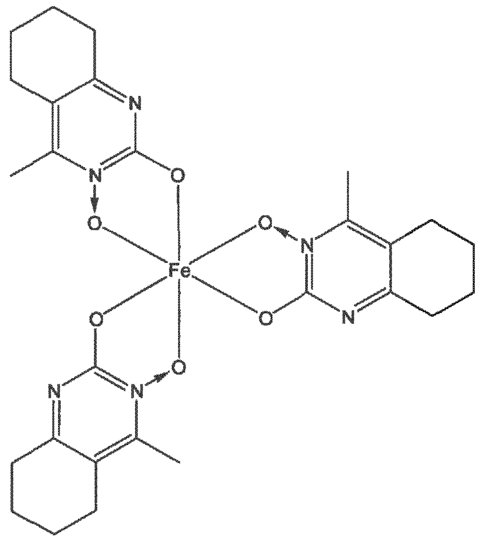

55 mmol (14.3 g) 4-Methyl-5,6,7,8-tetrahydrochinazolin-2-ol 3-oxid Hydrochlorid wurden in 30 ml Wasser gelöst und 18.3 mmol (4.95 g) in 5 ml Wasser gelöstes $FeCl_3$*$6H_2O$ zugegeben. Die Lösung wurde mit 110.7 ml 1 M NaOH auf pH 6.19 eingestellt und 0.5 h nachgerührt. Das Produkt wurde abfiltriert, mit Wasser gewaschen und bei 50°C im Vakuumtrockenschrank getrocknet. Man erhielt 10.7 g der Titelverbindung.
IR (in Substanz, $cm^{-1}$): 2932, 2859, 1586, 1510, 1446, 1421, 1377, 1348, 1308, 1267, 1229, 1189, 1168, 1100, 1079, 1057, 1030, 969, 889, 846, 824, 762, 704, 654, 614, 571, 507.
CHN-Elementaranalyse: C, 53.25; H, 5.49; N, 13.76.
Fe-Gehalt: 9.03% [m/m]
Chlorid-Gehalt: 0.0% [m/m]

**Beispiel 12**

**Tris-(4-(Propan-2-yl)pyrimidin-2-ol 1-oxid)-eisen(III)-Komplex**

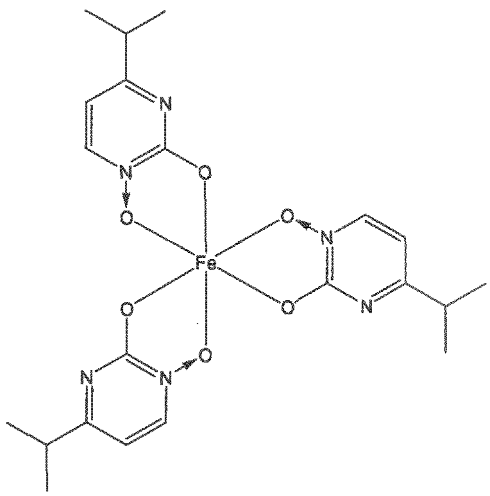

9.76 mmol (1.86 g) 4-(Propan-2-yl)pyrimidin-2-ol 1-oxid Hydrochlorid wurden in 5 ml Wasser gelöst und 3.25 mmol (0.88 g) in 2 ml Wasser gelöstes $FeCl_3*6H_2O$ zugegeben. Die Lösung wurde mit 18.5 ml 1 M NaOH auf pH 6.04 eingestellt und 0.5 h nachgerührt. Das Produkt wurde abfiltriert, mit Wasser gewaschen und bei 50°C im Vakuumtrockenschrank getrocknet. Man erhielt 1.64 g der Titelverbindung.
IR (in Substanz, $cm^{-1}$): 3071, 2965, 2930, 2871, 1594, 1540, 1510, 1467, 1428, 1373, 1309, 1239, 1204, 1152, 1132, 1108, 1049, 970, 931, 881, 834, 809, 777, 733, 712, 645, 599, 552, 514.
CHN-Elementaranalyse: C, 47.53; H, 5.01; N, 15.84.
Fe-Gehalt: 10.86% [m/m] (ICP)
Chlorid-Gehalt: 0.60% [m/m]

**Beispiel 13**

**Tris-(4-ethyl-6-methylpyrimidin-2-ol 1-oxid)-eisen(III)-Komplex**

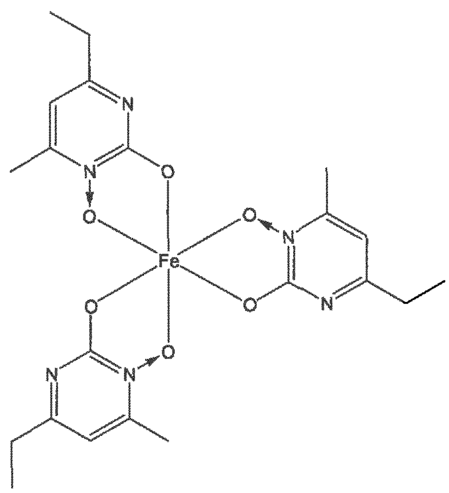

3.51 mmol (0.69 g) 4-Ethyl-6-methylpyrimidin-2-ol 1-oxid Hydrochlorid wurden in 3 ml Wasser gelöst und 1.17 mmol (0.316 g) $FeCl_3*6H_2O$ zugegeben. Die Lösung wurde mit 6.975 ml 1 M NaOH auf pH 6.37 eingestellt und 0.5 h nachgerührt. Das Produkt wurde abfiltriert, mit Wasser gewaschen und bei 50°C im Vakuumtrockenschrank getrocknet. Man erhielt 0.63 g (92% Fe-Ausbeute) der Titelverbindung.
IR (in Substanz, $cm^{-1}$): 2970, 2936, 2876, 1750, 1685, 1601, 1552, 1511, 1462, 1441, 1404, 1386, 1362, 1308, 1231, 1196, 1154, 1106, 1056, 1034, 984, 846, 807, 790, 770.
CHN-Elementaranalyse: C, 42.43; H, 5.43; N, 14.03.
Fe-Gehalt: 9.5% [m/m]

**Beispiel 14**

**Tris-(5-ethyl-4,6-dimethylpyrimidin-2-ol 1-oxid)-eisen(III)-Komplex**

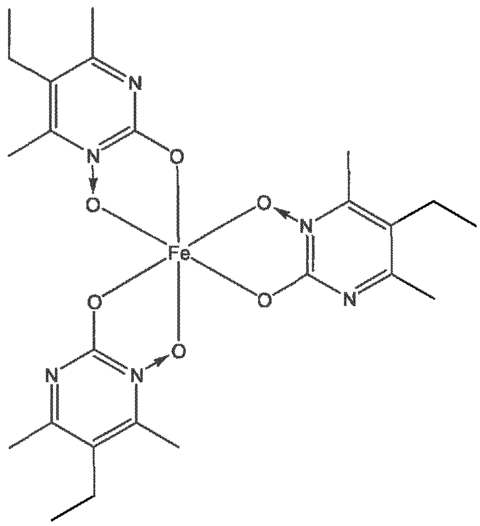

6.68 mmol (1.44 g) 5-Ethyl-4,6-dimethylpyrimidin-2-ol 1-oxid Hydrochlorid (Yamaguchi et al., J. Inorg. Biochemistry 2006, 100, 260-269) und 2.23 mmol (0.485 g) $FeCl_3*6H_2O$ wurden in 7 ml Wasser gelöst. Die Lösung wurde mit 1 M NaOH auf pH 6.0 eingestellt und 0.5 h nachgerührt. Das Produkt wurde abfiltriert, mit Wasser gewaschen und bei 50°C im Vakuumtrockenschrank getrocknet. Man erhielt 0.98 g (75% Fe-Ausbeute) der Titelverbindung.
IR (in Substanz, $cm^{-1}$): 2965, 1589, 1512, 1450, 1375, 1228, 1181, 1100, 1057, 1011,958,863,768,716,685,664.
CHN-Elementaranalyse: C, 49.67; H, 5.83; N, 14.52.
Fe-Gehalt: 9.54% [m/m]
Chlorid-Gehalt: 0.0% [m/m]

**Beispiel 15**

**Tris-(4-tert-butylpyrimidin-2-ol 1-oxid)-eisen(III)-Komplex**

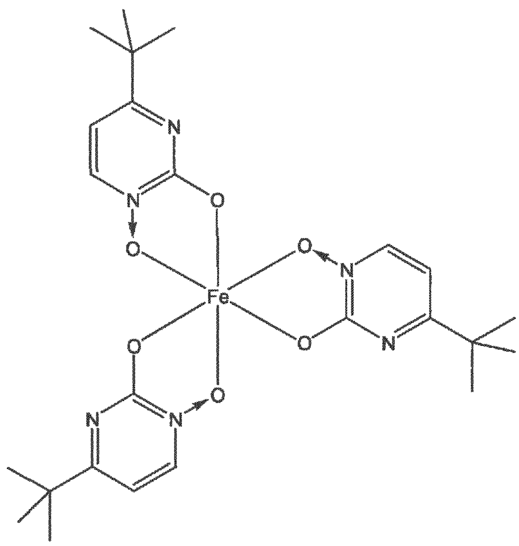

35.5 mmol (7.65 g) 4-tert-Butylpyrimidin-2-ol 1-oxid Hydrochlorid wurden in 15 ml Wasser gelöst und 11.84 mmol (3.20 g) $FeCl_3*6H_2O$ zugegeben. Die Suspension wurde mit 69.4 ml 1 M NaOH auf pH 6.1 eingestellt und 0.5 h nachgerührt. Das Produkt wurde abfiltriert, mit Wasser gewaschen und bei 50°C im Vakuumtrockenschrank getrocknet. Man erhielt 6.44 g (99% Fe-Ausbeute) der Titelverbindung.
IR (in Substanz, $cm^{-1}$):2965, 1598, 1535, 1506, 1477, 1419, 1364, 1339, 1273, 1238, 1217, 1159, 1122, 1025, 967, 829, 810, 779, 717, 701,
Fe-Gehalt: 10.15% [m/m]
Chlorid-Gehalt: 0.65% [m/m]

**Beispiel 16**

**Tris-(5,6-Dimethylpyrimidin-2-ol 1-oxid)-eisen(III)-Komplex**

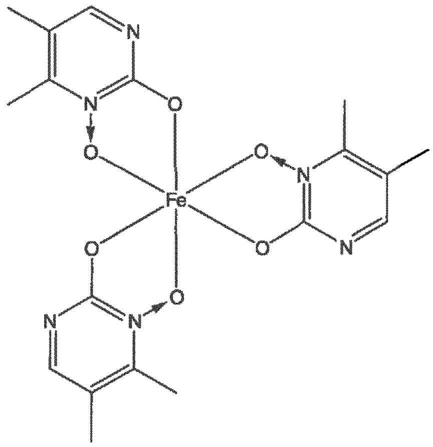

14.4 mmol (2.93 g) 5,6-Dimethylpyrimidin-2-ol Hydrochlorid wurden in 20 ml Wasser gelöst und 4.6 mmol (0.76 g) in 10 ml Wasser gelöstes $FeCl_3$ zugegeben. Die Lösung wurde mit 26.6 ml 1 M NaOH auf pH 5.8 eingestellt und 0.5 h nachgerührt. Das Produkt wurde abfiltriert, mit Wasser gewaschen und bei 50°C im Vakuumtrockenschrank getrocknet. Man erhielt 2.22 g (92% Fe-Ausbeute) der Titelverbindung.
IR (in Substanz, $cm^{-1}$): 3409, 1613, 1518, 1443, 1403, 1361, 1241, 1221, 1202, 1177, 1094, 1007, 880, 762, 714.
CHN-Elementaranalyse: C, 40.77; H, 4.95; N, 15.85.
Fe-Gehalt: 10.68% [m/m]
Chlorid-Gehalt: 1.1 % [m/m]

**Beispiel 17**

**Tris-(6-n-propylpyrimidin-2-ol 1-oxid)-eisen(III)-Komplex**

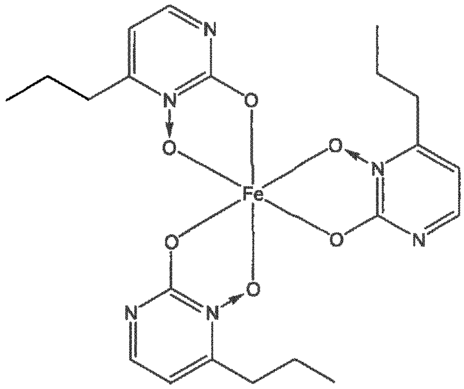

15 mmol (2.97 g) 6-n-Propylpyrimidin-2-ol 1-oxid Hydrochlorid und 5 mmol (1.334 g) $FeCl_3$*$6H_2O$ wurden in 42 ml Wasser und 18 ml Ethanol gelöst und auf 50 °C temperiert. Es wurde 30% Natronlauge auf pH 5.2 zugegeben und die Suspension auf Raumtemperatur abgekühlt. Das Produkt wurde abfiltriert, mit Wasser gewaschen und bei 50°C im Vakuumtrockenschrank getrocknet. Man erhielt 2.2 g (85% Fe-Ausbeute) der Titelverbindung.
IR (in Substanz, $cm^{-1}$): 3072, 2961, 1594, 1541, 1507, 1460, 1426, 1380, 1337, 1246, 1141, 1108, 1088, 979, 870, 838, 799, 768, 733, 691.
Fe-Gehalt: 10.74% [m/m]
Chlorid-Gehalt: 0.0% [m/m]

**Beispiel 18**

**Tris-(4-(ethylamino)pyrimidin-2-ol 1-oxid)-eisen(III)-Komplex**

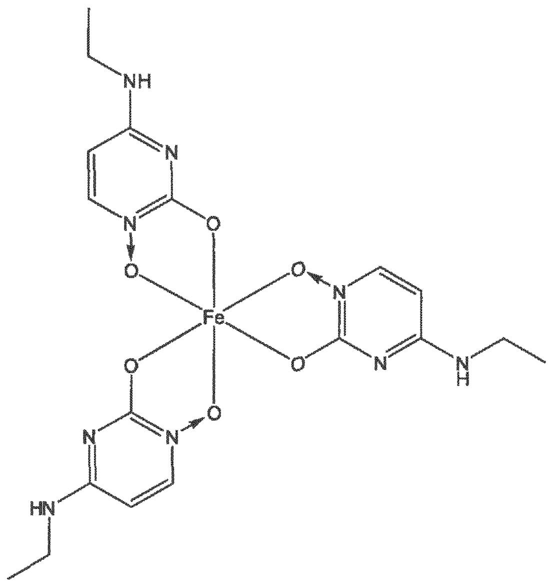

3 mmol (0.602 g) 4-(Ethylamino)pyrimidin-2-ol 1-oxid Hydrochlorid (Yamaguchi et al., J. Inorg. Biochemistry 2006, 100, 260-269) wurden in 20 ml Wasser gelöst und 1 mmol (0.27 g) in 2 ml Wasser gelöstes $FeCl_3$*$6H_2O$ zugegeben. Die Lösung wurde mit 1 M NaOH auf pH 5.7 eingestellt und 15 min nachgerührt. Das Produkt wurde abfiltriert, mit Wasser gewaschen und bei 50°C im Vakuumtrockenschrank getrocknet. Man erhielt 0.43 g (85% Fe-Ausbeute) der Titelverbindung.
IR (in Substanz, $cm^{-1}$): 3287, 2975, 1620, 1588, 1531, 1490, 1448, 1381, 1344, 1283, 1254, 1178, 1156, 1096, 1066, 1014, 826, 784, 754, 708.
Fe-Gehalt: 11.03% [m/m]
Chlorid-Gehalt: 0.47% [m/m]

**PHARMAKOLOGISCHE TESTMETHODE:**

Die hervorragenden Fe-Utilisationen, die durch die erfindungsgemäßen Fe-Komplexe, erreicht werden können, wurden durch folgendes Mäuse-Modell gemessen.

Männliche NMRI (SPF)-Mäuse (ca. 3 Wochen alt) wurden ca. 3 Wochen mit eisenarmer Diät (ca. 5 ppm Eisen) gefüttert. Dann wurde ihnen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). Die Utilisation am Tag 15 wurde berechnet aus der Hämoglobinzunahme und der Körpergewichtszunahme nach der Formel

$$\text{Utilisation (\%)} = \frac{\Delta \text{Eisenutilisation} * 100}{\text{Fe Dos.}} = \frac{(\text{Fe ut.} - \text{Fe ut.Control}) * 100}{\text{Fe Dos.}}$$

$$= [(Hb_{2(3)} * BW_{9(14)} - Hb_1 * BW_4) * 0{,}07 * 0{,}0034 - (Hb_{2(3)\ Control} * BW_{9(14)\ Control} - Hb_{1\ Control} * BW_{4\ Control}) * 0{,}07 * 0{,}0034)] * 100 / \text{Fe Dos.}$$

$$= [(Hb_{2(3)} * BW_{9(14)} - Hb_1 * BW_4) * 0{,}000238 - (Hb_{2(3)\ Control} * BW_{9(14)\ Control} - Hb_{1\ Control} * BW_{4\ Control}) * 0{,}000238] * 100 / \text{Fe Dos.}$$

$$= (Hb_{2(3)} * BW_{9(14)} - Hb_1 * BW_4 - Hb_{2(3)\ Control} * BW_{9(14)\ Control} + Hb_{1\ Control} * BW_{4\ Control}) * 0{,}0238 / \text{Fe Dos.}$$

| | |
|---|---|
| 0.07 | = Faktor für 70 ml Blut per kg Körpergewicht (BW) |
| 0.0034 | = Faktor für 0,0034 g Fe/g Hb |
| $Hb_1$ | = Hämoglobin-Wert (g/l) am Tag 1 |
| $Hb_{2(3)}$ | = Hämoglobin-Wert (g/l) am Tag 8 (or 15) |
| $BW_4$ | = Körpergewicht (g) am Tag 1 |
| $BW_{9(14)}$ | = Körpergewicht (g) am Tag 8 (or 15) |
| $Hb_{1\ Control}$ | = Durchschnitts-Hämoglobinwert (g/l) am Tag 1 in der Kontrollgruppe, |
| $Hb_{2(3)\ Control}$ | = Durchschnitts-Hämoglobinwert (g/l) am Tag 8 (oder 15) in der Kontrollgruppe, |
| $BW_{4\ Control}$ | = Durchschnittskörpergewicht (g) am Tag 1 in der Kontrollgruppe, |
| $BW_{9(14)\ Control}$ | = Durchschnittskörpergewicht (g) am Tag 8 (oder 15) in der Kontrollgruppe, |
| Fe Dos. | = Gesamtes verabreichtes Eisen (mg Fe) während 5 oder 10 Tage, |
| Fe ut. | = $(Hb_{2(3)} * BW_{9(14)} - Hb_1 * BW_4) * 0.07 * 0.0034$ (mg Fe) |
| Δ Utilisation | = Fe tot. utilisiert (Untersuchte Gruppe) - Fe ut. Kontrollgruppe, utilisiert aus Nahrung, (mg Fe). |

Die nachfolgende Tabelle zeigt die Ergebnisse:

Tabelle 1 - Eisenutilisation:

| Beispiel-Nr. | Utilisation n 15 d (abs. %) |
|---|---|
| 1 | 89 |
| 2 | 80 |
| 3 | 74 |
| 4 | 76 |
| 5 | Nicht bestimmt |
| 6 | 70 |
| 7 | 64 |
| 8 | 82 |
| 9 | 69 |
| 10 | 70 |

(fortgesetzt)

| Beispiel-Nr. | Utilisation n 15 d (abs. %) |
|---|---|
| 11 | 74 |
| 12 | 55 |
| 16 | 57 |
| Vergleichsbeispiel* | 25 |

***Vergleichsbeispiel:** Zum Vergleich wurde die Tris(pyridinon-2-ol 1-oxid)-Eisen(III)-Komplexverbindung der Formel:

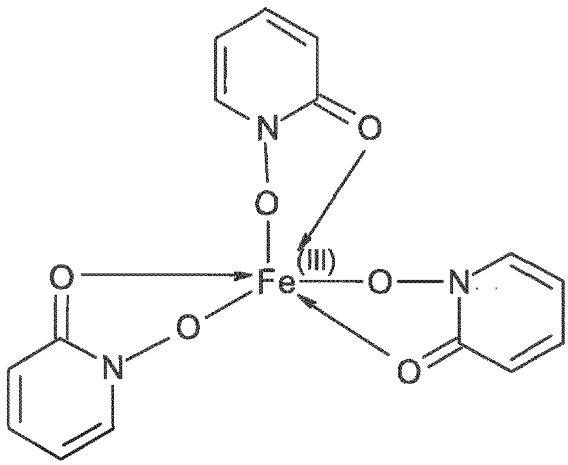

analog zur EP 0138420 hergestellt und getestet, um den Einfluß des heterocyclischen Grundkörpers zu belegen. EP 0138420 offenbart in Beispiel 7 lediglich Tris(pyridinon-2-ol 1-oxid)-Eisen(III)-Komplexverbindungen, die am Pyridinring einen weiteren Substituenten tragen. Die unsubstituierte, hier zum Vergleich herangezogene Tris(pyridinon-2-ol 1-oxid)-Eisen(III)-Komplexverbindung wird dort nicht offenbart. Wie die Ergebnisse in vorstehender Tabelle zeigen, weist die entsprechende erfindungsgemäße Pyrimidin-Verbindung des Beispiels 1 gegenüber der Pyridin-Vergleichsverbindung analog EP 0138420 eine deutlich verbesserte Eisen-Utilisation auf.

## Patentansprüche

**1.** Eisen(III)-Komplexverbindungen zur Verwendung als Arzneimittel, enthaltend mindestens einen Liganden der Formel (I):

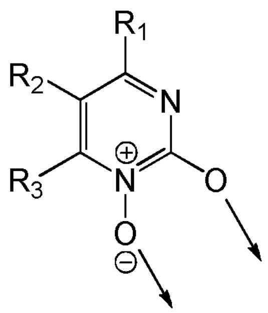

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl,
- Halogen,

- gegebenenfalls substituiertem Alkoxy,
- gegebenenfalls substituiertem Aryl,
- gegebenenfalls substituiertem Alkoxycarbonyl,
- gegebenenfalls substituiertem Amino, und
- gegebenenfalls substituiertem Aminocarbonyl ist, oder

$R_1$ und $R_2$ oder $R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann,
oder pharmazeutisch verträgliche Salze davon.

**2.** Eisen(III)-Komplexverbindungen zur Verwendung nach Anspruch 1, enthaltend mindestens einen Liganden der Formel (I):

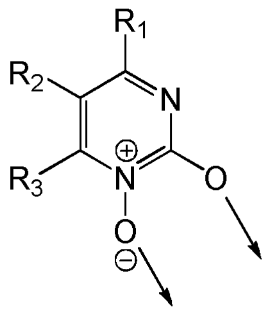

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl,
- Halogen,
- gegebenenfalls substituiertem Alkoxy,
- gegebenenfalls substituiertem Aryl,
- gegebenenfalls substituiertem Alkoxycarbonyl, und
- gegebenenfalls substituiertem Aminocarbonyl ist, oder

$R_1$ und $R_2$ oder $R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann,
oder pharmazeutisch verträgliche Salze davon.

**3.** Eisen(III)-Komplexverbindungen zur Verwendung nach Anspruch 1 oder 2, enthaltend mindestens einen Liganden der Formel (I):

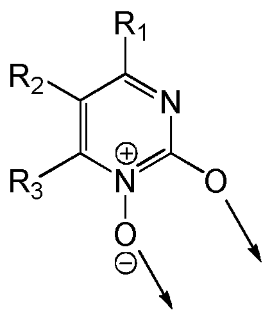

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl, und
- Halogen, oder

$R_1$ und $R_2$ oder $R_2$ und $R_3$ gemeinsam mit den Kohlenstoffatomen, an die sie gebunden sind, einen 5- oder 6-gliedrigen carbocyclischen Ring bilden,
oder pharmazeutisch verträgliche Salze davon.

**4.** Eisen(III)-Komplexverbindungen zur Verwendung nach einem der Ansprüche 1 bis 3, enthaltend mindestens einen Liganden der Formel (I):

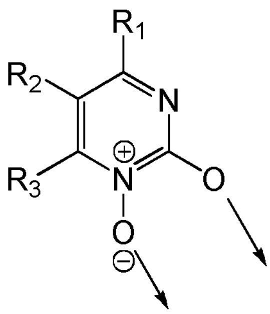

(I)

worin
die Pfeile jeweils eine koordinative Bindung zu einem oder verschiedenen Eisenatomen darstellen,
$R_1$, $R_2$, $R_3$ gleich oder verschieden sind und ausgewählt werden aus der Gruppe, die besteht aus Wasserstoff, Alkyl, das gegebenenfalls durch Alkoxy substituiert sein kann, und Halogen besteht, oder
$R_1$ und $R_2$ oder $R_2$ und $R_3$ bilden zusammen eine Propylen ($-CH_2-CH_2-CH_2-$), Butylen ($-CH_2-CH_2-CH_2-CH_2-$), Azabutylen oder Oxabutylen-Gruppe,
oder pharmazeutisch verträgliche Salze davon.

**5.** Eisen(III)-Komplexverbindungen zur Verwendung nach einem der Ansprüche 1 bis 4, worin $R_1$, $R_2$, $R_3$ gleich oder verschieden sind und ausgewählt werden aus der Gruppe, die besteht aus Wasserstoff und Alkyl, mit der Maßgabe, dass mindestens einer der Substituenten $R_1$, $R_2$, und $R_3$ Alkyl ist.

**6.** Eisen(III)-Komplexverbindungen zur Verwendung nach Anspruch 5, worin $R_2$ Wasserstoff ist, und $R_1$ und $R_3$ jeweils gleich oder verschieden sind und aus Alkyl ausgewählt sind.

**7.** Eisen(III)-Komplexverbindungen zur Verwendung nach einem der Ansprüche 1 bis 6, welche die folgende Formel aufweisen:

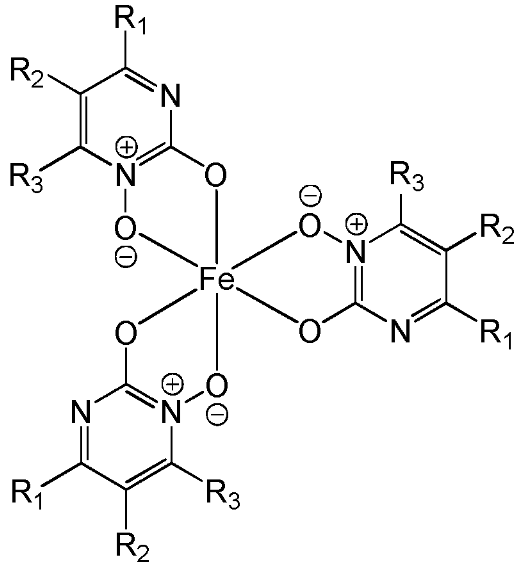

,

worin $R_1$, $R_2$, $R_3$ jeweils gleich oder verschieden sein können und wie oben definiert sind, und pharmazeutisch verträgliche Salze davon.

**8.** Eisen(III)-Komplexverbindungen zur Verwendung nach einem der Ansprüche 1 bis 7 zur Verwendung in der Behandlung und Prophylaxe von Eisenmangelerscheinungen und Eisenmangelanämien sowie der damit einhergehenden Symptome.

**9.** Eisen(III)-Komplexverbindungen zur Verwendung nach Anspruch 8, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

**10.** Eisen(III)-Komplexverbindungen zur Verwendung nach Anspruch 8 oder 9 zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

**11.** Eisen(III)-Komplexverbindungen zur Verwendung nach einem der Ansprüche 1 bis 7, worin die Eisen(III)-Komplexverbindung oral verabreicht wird.

**12.** Eisen(III)-Komplexverbindungen zur Verwendung nach Anspruch 11, worin eine feste Formulierung, wie Pillen, Tabletten, magensaftresistente Tabletten, Filmtabletten, Schichttabletten, Retardformulierung, Depotformulierung, Dragees, Zäpfchen, Granulat, Suppositorien, Mikrokapseln, Mikroformulierungen, Nanoformulierungen, Kapseln, magensaftresistente Kapseln und Pulver oder eine flüssige Formulierung, einschließlich einer trinkbaren Formulierung, wie Sirup, Elixier, Lösung, Suspension, Saft, verabreicht wird.

**13.** Arzneimittel, enthaltend Eisen(III)-Komplexverbindungen wie in einem der Ansprüche 1 bis 7 definiert.

**14.** Arzneimittel, enthaltend Eisen(III)-Komplexverbindungen wie in einem der Ansprüche 1 bis 7 definiert sowie mindestens einen physiologisch verträglichen Träger bzw. Exzipienten.

**15.** Zusammensetzung enthaltend Eisen(III)-Komplexverbindungen, wie in einem der Ansprüche 1 bis 7 definiert, in Kombination mit mindestens einem weiteren Arzneimittel, welches auf den Eisenmetabolismus wirkt.

## Claims

**1.** Iron(III) complex compounds for use as a medicament, containing at least one ligand of the formula (I):

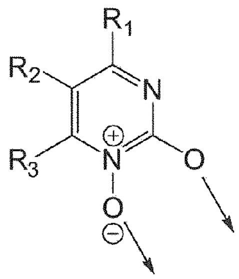

(I)

wherein
the arrows respectively represent a coordinate bond to one or different iron atoms, and
$R_1$, $R_2$, $R_3$ may be the same or different and are selected from the group consisting of:

- hydrogen,
- optionally substituted alkyl,
- halogen,
- optionally substituted alkoxy,
- optionally substituted aryl,
- optionally substituted alkoxycarbonyl,
- optionally substituted amino, and
- optionally substituted aminocarbonyl or

$R_1$ and $R_2$ or $R_2$ and $R_3$ together with the carbon atoms to which they are bonded, form an optionally substituted saturated or unsaturated 5- or 6-membered ring, which may optionally contain one or more heteroatoms, or pharmaceutically acceptable salts thereof.

**2.** Iron(III) complex compounds for use according to claim 1, containing at least one ligand of the formula (I):

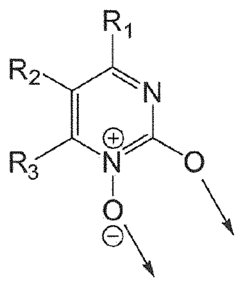

(I)

wherein
the arrows respectively represent a coordinate bond to one or different iron atoms, and
$R_1$, $R_2$, $R_3$ may be the same or different and are selected from the group consisting of:

- hydrogen,
- optionally substituted alkyl,
- halogen,
- optionally substituted alkoxy,
- optionally substituted aryl,
- optionally substituted alkoxycarbonyl, and
- optionally substituted aminocarbonyl or

$R_1$ and $R_2$ or $R_2$ and $R_3$ together with the carbon atoms to which they are bonded, form an optionally substituted saturated or unsaturated 5- or 6-membered ring, which may optionally contain one or more heteroatoms, or pharmaceutically acceptable salts thereof.

**3.** Iron(III) complex compounds for use according to claim 1 or 2, containing at least one ligand of the formula (I):

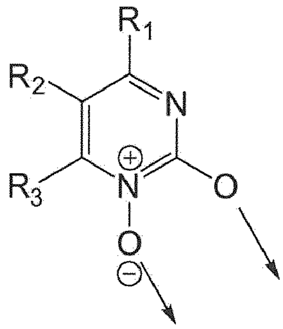

(I)

wherein
the arrows respectively represent a coordinate bond to one or different iron atoms, and
$R_1$, $R_2$, $R_3$ may be the same or different and are selected from the group consisting of:

- hydrogen
- optionally substituted alkyl, and
- halogen or

$R_1$ and $R_2$ or $R_2$ and $R_3$ together with the carbon atoms to which they are bonded form a 5- or 6-membered carbocyclic ring,
or pharmaceutically acceptable salts thereof.

**4.** Iron(III) complex compounds according to any one of claims 1 to 3, containing at least one ligand of the formula (I):

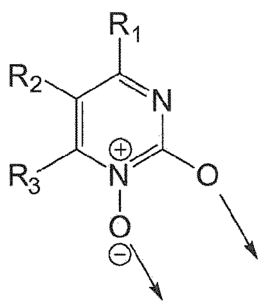

(I)

wherein
the arrows respectively represent a coordinate bond to one or different iron atoms,
$R_1$, $R_2$, $R_3$ may be the same or different and are selected from the group consisting of hydrogen, alkyl which may optionally be substituted by alkoxy, and halogen, or
$R_1$ and $R_2$ or $R_2$ and $R_3$ form together a propylene ($-CH_2-CH_2-CH_2-$), a butylene ($-CH_2-CH_2-CH_2-CH_2-$), azabutylene

or oxabutylene group,
or pharmaceutically acceptable salts thereof.

5. Iron(III) complex compounds according to any one of claims 1 to 4, wherein $R_1$, $R_2$, $R_3$ are identical or different and are selected from the group consisting of hydrogen and alkyl, with the proviso that at least one of the substituents $R_1$, $R_2$, and $R_3$ is alkyl.

6. Iron(III) complex compounds for the use according to claim 5, wherein $R_2$ is hydrogen, and $R_1$ and $R_3$ are each the same or different and are selected from alkyl.

7. Iron(III) complex compounds for use according to any of the claims 1 to 6 of the formula:

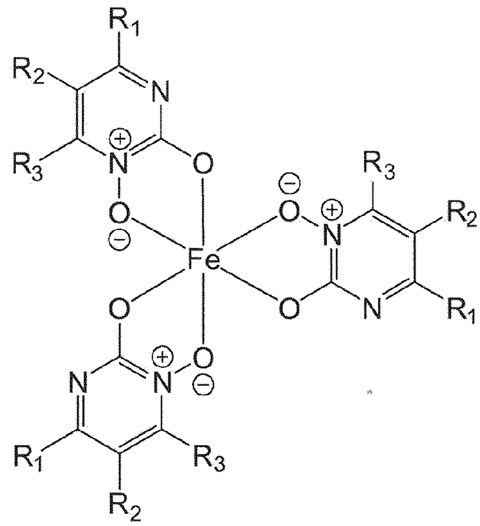

wherein $R_1$, $R_2$, $R_3$ may be the same or different and are defined as above, and pharmaceutically acceptable salts thereof.

8. Iron(III) complex compounds according to any of the claims 1 to 7 for use in the treatment and prophylaxis of iron deficiency symptoms and iron deficiency anemias and the symptoms associated therewith.

9. Iron(III) complex compounds for use according to claim 8, wherein the symptoms include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

10. Iron(III) complex compounds for use according to claim 8 for the treatment of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage (e.g. due to ulcers, carcinoma, hemorrhoids, inflammatory disorders, taking of acetylsalicylic acid), iron deficiency anemia caused by menstruation, iron deficiency anemia caused by injuries, iron deficiency anemia due to psilosis (sprue), iron deficiency anemia due to reduced dietary iron uptake, in particular in selectively eating children and adolescents, immunodeficiency caused by iron deficiency anemia, brain function impairment caused by iron deficiency anemias, restless leg syndrome caused by iron deficiency anemias, iron deficiency anemias in the case of cancer, iron deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CDK 3-5; chronic kidney diseases stage 3-5), iron deficiency anemias triggered by chronic inflammation (ACD), iron deficiency anemias in the case of rheumatoid arthritis (RA), iron deficiency anemias in the case of systemic lupus erythematosus (SLE) and iron deficiency anemias in the case of inflammatory bowel diseases (IBD).

11. Iron(III) complex compounds for use according to any one of the claims 1 to 7, wherein the iron(III) complex compound

is administered orally.

**12.** Iron(III) complex compounds for use according to claim 11, wherein a solid formulation such as pills, tablets, enteric-coated tablets, film tablets, layer tablets, sustained release formulations, depot formulations, dragees, suppositories, granulates, microcapsules, microformulations, nanoformulations, capsules, enteric-coated capsules and powders, or a liquid formulation including a drinkable formulation such as a syrup, elixir, solution, suspension, juice is administered.

**13.** Medicament, containing iron(III) complex compounds as defined in any one of claims 1 to 7.

**14.** Medicament, containing iron(III) complex compounds as defined in any one of claims 1 to 7 and at least one physiological compatible carrier or excipient.

**15.** Composition containing iron(III) complex compounds as defined in any one of claims 1 to 7, in combination with at least one further medicament which acts on the iron metabolism.

## Revendications

**1.** Composés à base de complexes de fer(III) pour être utilisés en tant que médicaments, contenant au moins un ligand de la formule (I);

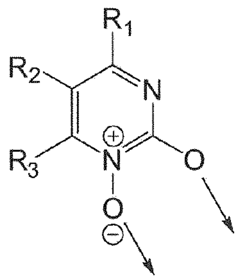

(I)

dans laquelle
les flèches représentent respectivement une liaison de coordination à un ou différents atomes de fer, et
$R_1$, $R_2$, $R_3$ peuvent être identiques ou différents et sont choisis parmi le groupe consistant en:

- hydrogène,
- alkyle éventuellement substitué,
- halogène,
- alkoxy éventuellement substitué,
- aryle éventuellement substitué,
- alkoxycarbonyle éventuellement substitué,
- amino éventuellement substitué, et
- aminocarbonyle éventuellement substitué, ou

$R_1$ et $R_2$ ou $R_2$ et $R_3$ conjointement aux atomes de carbone auxquels ils sont liés, forment un cycle de 5 ou 6 chaînons éventuellement substitués, saturés ou insaturés, qui peuvent éventuellement contenir un ou plusieurs hétéroatomes, ou les sels pharmaceutiquement acceptables de ceux-ci.

**2.** Composés à base de complexes de fer(III) pour une utilisation selon la revendication 1 , contenant au moins un ligand de la formule (I);

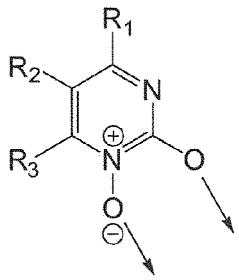

(I)

dans laquelle
les flèches représentent respectivement une liaison de coordination a un ou différents atomes de fer, et
$R_1$, $R_2$, $R_3$ peuvent être identiques ou différents et sont choisis parmi le groupe consistant en:

- hydrogène,
- alkyle éventuellement substitué,
- halogène,
- alkoxy éventuellement substitué,
- aryle éventuellement substitué,
- alkoxycarbonyle éventuellement substitué, et
- aminocarbonyle éventuellement substitué, ou

$R_1$ et $R_2$ ou $R_2$ et $R_3$, conjointement aux atomes de carbone auxquels ils sont liés, forment un cycle de 5 ou 6 chaînons éventuellement substitués, saturés ou insaturés, qui peuvent éventuellement contenir un ou plusieurs hétéroatomes, ou les sels pharmaceutiquement acceptables de ceux-ci.

**3.** Composés à base de complexes de fer(III) pour une utilisation selon la revendication 1 ou 2, contenant au moins un ligand de la formule (I):

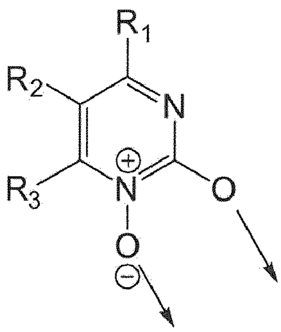

(I)

dans laquelle
les flèches représentent respectivement une liaison de coordination à un ou différents atomes de fer, et
$R_1$, $R_2$, $R_3$ peuvent être identiques ou différents et sont choisis parmi le groupe consistant en:

- hydrogène,
- alkyle éventuellement substitué,
- halogène ou

$R_1$ et $R_2$ ou $R_2$ et $R_3$, conjointement aux atomes de carbone auxquels ils sont liés, forment un cycle carbocyclique de 5 ou 6 chaînons,
ou les sels pharmaceutiquement acceptables de ceux-ci.

**4.** Composés à base de complexes de fer(III) pour une utilisation selon la revendication 1 à 3, contenant au moins un ligand de la formule (I):

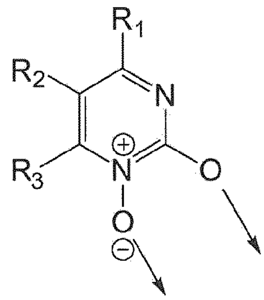

(I)

dans laquelle
les flèches représentent respectivement une liaison de coordination à un ou différents atomes de fer,
$R_1$, $R_2$, $R_3$ peuvent être identiques ou différents et sont choisis parmi le groupe consistant en hydrogène, alkyle qui peut être éventuellement substitué par alkoxy, et halogène,
$R_1$ et $R_2$ ou $R_2$ et $R_3$, forment ensemble un groupe propylene ($-CH_2-CH_2-CH_2-$), butylène ($-CH_2-CH_2-CH_2-CH_2-$), azabutylene ou oxabutylene,
ou les sels pharmaceutiquement acceptables de ceux-ci.

**5.** Composés à base de complexes de fer(III) pour une utilisation selon la revendication 1 à 4, , dans lesquels $R_1$, $R_2$, $R_3$ sont identiques ou différents et sont choisis parmi le groupe consistant en hydrogène et alkyle, à la condition qu'au moins l'un des substituants $R_1$, $R_2$ et $R_3$ soit alkyle.

**6.** Composés à base de complexes de fer(III) pour une utilisation selon la revendication 5, dans lesquels $R_2$ est hydrogène, et $R_1$ et $R_3$ sont chacun les mêmes ou différents et sont choisis parmi l'alkyle.

**7.** Composés à base de complexes de fer(III) pour une utilisation selon l'une quelconque des revendications 1 à 6 de la formule:

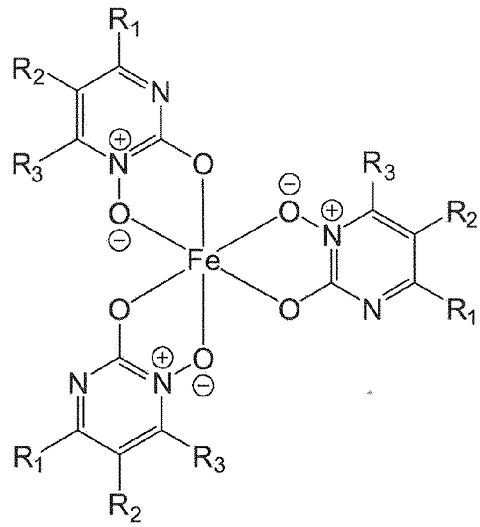

dans laquelle $R_1$, $R_2$, $R_3$ peuvent être identiques ou différents et sont définis comme ci-dessus, et les sels pharmaceutiquement acceptables de ceux-ci.

**8.** Composés à base de complexes de fer(III) selon l'une quelconque des revendications 1 à 7 pour utilisation dans le traitement et la prévention de carences en fer et d'anémies dues à une carence en fer et les symptômes qui y sont associés.

**9.** Composés à base de complexes de fer(III) pour une utilisation selon la revendication 8, dans lesquels les symptômes comprennent: la fatigue, manque d'énergie, mauvaise concentration, faible efficacité cognitive, difficultés à trouver

les mots justes, manque de mémoire, pâleur anormale, irritabilité, accélération du rythme cardiaque (tachycardie), langue douloureuse ou gonflée, rate hypertrophiée, désir pour des aliments étranges (pica), maux de tête, manque d'appétit, prédisposition accrue aux infections, humeurs depressives.

**10.** Composés à base de complexes de fer(III) pour l'utilisation selon la revendication 8 ou 9, pour le traitement de l'anémie due à une carence en fer chez les femmes enceintes, l'anémie due à une carence en fer latente chez les enfants et les adolescents, l'anémie due à une carence en fer causée par des anomalies gastro-intestinales, l'anémie due à une carence en fer due à la perte de sang, telle que l'hémorragie gastro-intestinale (par exemple en raison d'ulcères, carcinomes, hémorroïdes, maladies inflammatoires, la prise d'acide acétylsalicylique), l'anémie due à une carence en fer causée par la menstruation, l'anémie due à une carence en fer causée par des blessures, l'anémie due à une carence en fer causée par le psilosis (sprue), l'anémie due à une carence en fer causée par l'absorption réduite de fer alimentaire, en particulier chez les enfants et les adolescents ayant une alimentation selective, l'immunodéficience causée par l'anémie due à une carence en fer, l'affaiblissement de la fonction cérébrale causé par des anémies dues à une carence en fer, le syndrome des jambes sans repos causé par des anémies dues à une carence en fer, les anémies dues à une carence en fer dans le cas du cancer, les anémies dues à une carence en fer causée par des chémothérapies, les anémies dues à une carence en fer déclenchée par l'inflammation (AI), les anémies dues à une carence en fer dans le cas d'insuffisance cardiaque congestive (ICC), les anémies de carence en fer dans le cas d'insuffisance rénale chronique stade 3-5 (CDK 3-5; maladies rénales chroniques stade 3-5), les anémies de carence en fer provoquées par l'inflammation chronique (ACD), les anémies de carence en fer dans le cas de l'arthrite rhumatoïde (AR), les anémies de carence en fer dans le cas du lupus érythémateux systémique (LES) et les anémies de carence en fer dans le cas des maladies inflammatoires des intestins (MII).

**11.** Composés à base de complexes de fer(III) pour l'utilisation selon l'une quelconque des revendications 1 à 7, où le composé à base de complexe de Fe(III) est administré par voie orale.

**12.** Composés à base de complexes de fer(III) pour l'utilisation selon la revendication 1 1 , dans lesquels une formulation solide telle que des pilules, des comprimés, des comprimés entérosolubles, des comprimés pellicules, des comprimés à couches, des formulations à libération prolongée, des formulations de dépôt, des dragées, des suppositoires, des granulés, des microcapsules, des microformulations, des nanoformulations, des capsules, des capsules entérosolubles et des poudres, ou une formulation liquide comprenant une formulation buvable telle qu'un sirop, un élixir, une solution, une suspension, un jus, est administrée.

**13.** Médicament, contenant des composés à base de complexes de fer(III) tels que définis dans l'une quelconque des revendications 1 à 7.

**14.** Médicament, contenant des composés à base de complexes de fer(III) tels que définis dans l'une quelconque des revendications 1 à 7 et au moins un support ou excipient physiologique compatible.

**15.** Composition contenant des composés à base de complexes de Fe(III) tels que définis dans l'une quelconque des revendications 1 a 7, en combinaison avec au moins un autre médicament qui agit sur le métabolisme du fer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- EP 159194 A
- EP 138420 A
- EP 107458 A
- WO 2006037449 A
- GB 842637 A
- EP 0138420 A


### In der Beschreibung aufgeführte Nicht-Patentliteratur


- **M.W. HENTZE.** Balancing acts: molecular control of mammalian iron metabolism. *Cell,* 2004, vol. 117, 285-297
- *Biometals,* 2009, vol. 22, 701-710
- *Bull. Chem. Soc. Jpn.,* 1993, vol. 66, 841-841
- *Reviews On Heteroatom Chemistry,* 1998, vol. 18, 87-118
- *J. Org. Chem.,* 1997, vol. 62, 3618-3624
- *J. Am. Chem. Soc.,* 1985, vol. 107, 6540-6546
- *Inorganica Chimica Acta,* 1987, vol. 135, 145-150
- *Tetrahedron,* 1967, vol. 23, 353-357
- **OHKANDA.** *Bull. Chem. Soc. Jpn.,* 1993, vol. 66, 841-847
- *Can. J. Chem.,* 1984, vol. 62, 1176-1180
- **T. HARAYAMA ; H. CHO ; Y. INUBUSHI.** *Chem. Pharm. Bull.,* 1978, vol. 26, 1201-1214
- **E.E. ROYALS ; K.C. BRANNOCK.** *J. Am. Chem. Soc.,* 1953, vol. 75, 2050-2053
- **M. YAMAGUCHI et al.** *J. Inorg. Biochemistry,* 2006, vol. 100, 260-269
- **YAMAGUCHI et al.** *J. Inorg. Biochemistry,* 2006, vol. 100, 260-269